# EUROPÄISCHE PATENTSCHRIFT

(11) **EP 3 881 772 B1**
(45) Veröffentlichungstag und Bekanntmachung des Hinweises auf die Patenterteilung: **27.11.2024**
(21) Anmeldenummer: 21160387.3
(22) Anmeldetag: 03.03.2021
(51) Int. Cl.: A61B 17/04

(54) **KNOCHENANKERELEMENT ZUM EINBRINGEN IN EINEN KNOCHEN UND/ODER FIXIEREN VON GEWEBE AN DEM KNOCHEN, KNOCHENANKERSYSTEM UND VERFAHREN ZUM KONFEKTIONIEREN EINES KNOCHENANKERSYSTEMS**
BONE ANCHOR ELEMENT FOR INSERTION INTO A BONE AND/OR FIXATION OF TISSUE TO THE BONE, BONE ANCHOR SYSTEM AND METHOD FOR PREPARING A BONE ANCHOR SYSTEM
ÉLÉMENT D'ANCRAGE OSSEUX PERMETTANT D'INSÉRER DANS UN OS ET/OU DE FIXER UN TISSU À L'OS, SYSTÈME D'ANCRAGE OSSEUX ET PROCÉDÉ DE FABRICATION D'UN SYSTÈME D'ANCRAGE OSSEUX

(30) Priorität: 17.03.2020 DE 102020107245
(43) Veröffentlichungstag der Anmeldung: 22.09.2021
(73) Patentinhaber: Karl Storz SE & Co. KG, 78532 Tuttlingen (DE)
(72) Erfinder: Biedermann, Hannes, 78532 Tuttlingen (DE); Fluri, Daniel, 2540 Grenchen (CH); Bouduban, Nicolas, 2503 Biel (CH)
(74) Vertreter: Weidner Stern Jeschke

(56) Entgegenhaltungen:
- EP-A1- 1 917 915
- EP-A2- 2 606 833
- US-A1- 2018 338 753
- US-A1- 2019 150 912

## Beschreibung

Die Erfindung betrifft ein Knochenankerelement zum Einbringen in einen Knochen und/oder Fixieren von Gewebe an dem Knochen, wobei das Knochenankerelement in einer Längsrichtung ein proximales Ende, einen proximalen Abschnitt, einen distalen Abschnitt und ein distales Ende aufweist, und der proximale Abschnitt außen einen profilierten Vorsprung zum Verhindern eines Abziehens des in den Knochen eingebrachten Knochenankerelementes und innen einen durchgehenden Hohlraum in der Längsrichtung mit einer proximalen Öffnung und einer distalen Öffnung aufweist, und der distale Abschnitt plattenförmig abgeflacht mit einer Plattenebene ausgebildet ist. Des Weiteren betrifft die Erfindung einen Einbringer zum Einbringen eines Knochenankerelementes in ein Bohrloch in einem Knochen, ein Knochenankersystem und ein Verfahren zum Konfektionieren eines Knochenankersystems.

Knochenankerelemente, auch Knochenanker genannt, werden im medizinischen Bereich eingesetzt, um von einem Knochen abgelöstes Gewebe, wie beispielsweise eine abgerissene Sehne, wieder am Knochen zu fixieren. Dazu weist das Knochenankerelement üblicherweise zumindest ein Loch oder eine Aufnahme zum Durchfädeln eines Fadens auf und das Knochenankerelement wird mit dem Faden beladen in den Knochen eingeschlagen oder eingeschraubt. Hierzu wird üblicherweise ein sogenannter Eintreiber oder Einbringer verwendet, welcher auf das proximale Ende des Knochenankerelementes aufgesetzt wird.

Abhängig von der Vernähtechnik stehen beispielsweise die beiden Fadenenden des im Knochenankerelement aufgenommenen Fadens am proximalen Ende des Knochenankerelementes heraus und werden längs des Eintreibers geführt und gehalten. Nach Eintreiben des Knochenankerelementes und Abnehmen des Eintreibers werden die beiden freien Fadenenden dazu genutzt, das abgelöste Gewebe unter Verknoten der Fadenenden am Knochen zu befestigen.

Alternativ wird üblicherweise bei einer bekannten knotenfreien Operationstechnik der Faden durch eine Öffnung des Knochenankerelementes hindurchgefädelt, anschließend eines der freien Fadenenden unter Verwendung einer Nadel durch das abgelöste Gewebe hindurchgestochen und dieses Fadenende dann wieder durch die Öffnung im Knochenankerelement in entgegengesetzter Richtung hindurchgefädelt. Folglich wird hierbei das abgelöste Gewebe mit einer Fadenschleife ohne einen Knoten am Gewebe fixiert, bei der die beiden Fadenenden am Knochenankerelement gehalten werden. Das Knochenankerelement wird in den Knochen eingebracht und durch Ziehen an den beiden freien Fadenenden das zu fixierende Gewebe an den Knochen herangezogen.

Aus der US 2006/0079904 A1 ist beispielsweise ein System mit zwei Knochenankern zur Gewebereparatur bekannt, wobei der erste Knochenanker an seinem proximalen Ende ein Einfädelloch aufweist, durch welches eine Fadenschleife geführt ist. Dieser erste Knochenanker wird in den Knochen eingeschraubt, die Fadenschleife durch eine abgelöste Sehne durchgezogen und anschließend wird das Fadenschlaufenende innerhalb einer mittigen Öffnung am distalen Ende des zweiten Knochenankers aufgenommen und dieser in den Knochen eingebracht. Nachteilig hierbei ist, dass zwei Knochenanker verwendet und somit zunächst zwei Bohrlöcher in den Knochen eingebracht werden müssen. Zudem muss die Fadenschleife des ersten Knochenankers im Körper mit dem zweiten Knochenanker durch den Operateur verbunden werden.

Die US 2012/0022588 A1 offenbart ebenfalls ein knotenloses Verfahren zum Befestigen eines Gewebes an einem Knochen, wobei eine Knochenschraube eine zentrale Bohrung zur Führung auf einer Einbringerstange aufweist und ein Anker eine Fadenschleife fängt, die durch das abgelöste Gewebe geführt ist. Zusätzlich kann eine zweite Fadenschleife durch das abgelöste Gewebe geführt sein, welche an ihrem entgegengesetzten Fadenschleifenende in einer am distalen Ende des Ankers angeordneten Öffnung aufgenommen und durch Einbringen der Knochenschraube im Knochen befestigt wird.

Die US 2016/0192924 A1 offenbart einen chirurgischen Nahtanker mit einem innenliegenden durchgehenden Hohlraum und einer sich distalseitig anschließenden Aussparung, welche um ein Nahtaufnahmeelement umgeben von Seitenwänden um dessen distale Spitze zur Aufnahme von zwei Nahtfäden geführt ist.

In der US 2009/0076544 A1 ist ein Nahtanker mit einem durchgehenden Hohlraum und zwei Durchbrüchen ausgebildet als eine Aussparung getrennt durch ein Fadenaufnahmeelement beschrieben, wobei das Fadenaufnahmeelement und die Durchbrüche von Seitenwänden umgeben sind und der Nahtanker eine spitz zulaufende distale Spitze zum Einbringen in einen Knochen aufweist.

Die DE 195 304 A1 offenbart einen kanülierten Greifer mit einem distalseitigen hohlen Lauf und proximalseitig einer Kappe mit einer Fluidsperre, wobei ein durch ein endständiges Auge mit einem Faden versehener, schraubenförmiger Fadenanker durch den kanülierten Greifer mittels einer Drehvorrichtung durch die Fluidsperre eingeführt wird.

Die WO 2019/123462 A1 offenbart ein medizinisches Implantat aufweisend eine Vielzahl von verstärkten, biologisch abbaubaren Faserbündeln, bei dem durch Löcher in den Wänden des medizinischen Implantats ein besseres Einwachsen in ein Gewebe erreicht wird.

Die EP 2 606 833 A2 beschreibt einen knotenlosen Nahtanker, welcher einen proximalen Abschnitt mit einem durchgehenden Hohlraum und anschließend einen distalen Abschnitt mit zwei sich gegenüberliegenden distalen Armen aufweist, wobei zwischen den beiden gegenüberliegenden distalen Armen innen ein Nahtsitzelement mit einer seitlichen, um dessen distalen Ende herumgeführten Aussparung angeordnet ist, und ein Zugfaden außen geführt um das Nahtsitzelement durch eine seitliche Öffnung in einer planaren Seitenwand nach außen zwischen der planaren Seitenwand und einer Knochenwand gleiten und gezogen werden kann.

Nachteilig bei bekannten Knochenankerelementen und/oder -systemen ist, dass diese zwei- oder mehrteilig ausgeführt sind und erst unter der Operation vom Operateur zusammengesetzt werden müssen. Des Weiteren ist nachteilig, dass die Beladung des Knochenankersystems vorgegeben und/oder vorkonfektioniert ist und bei bekannten Knochenankersystemen nicht flexibel interoperativ anpassbar und einsetzbar ist. Zudem erlaubt der jeweilige Knochenanker häufig nur eine knotenlose oder eine konventionelle Beladung. Somit steht nicht ein einziger Knochenanker zur Verfügung, welcher für verschiedene Operationstechniken und für eine flexible Beladung mit dem Nahtfaden bereitsteht.

Aufgabe der Erfindung ist es, den Stand der Technik zu verbessern.

Gelöst wird die Aufgabe durch ein Knochenankerelement gemäß dem Anspruch 1 und durch Verfahren gemäß dem Anspruch 18.

Folglich stellt das Knochenankerelement insbesondere mehrere unterschiedliche Möglichkeiten der Fadenführung bereit, von denen eine oder mehrere, die für eine jeweilige Anwendung besonders geeignet ist oder sind, beispielsweise bei einer Konfektionierung und/oder prä- oder intraoperativ von einem Benutzer gewählt werden kann oder können.

Somit wird ein Knochenankerelement bereitgestellt, welches konventionell mit einem Faden als Nahtmaterial vorbeladen und somit vorkonfektioniert ist und/oder welches vor und/oder während der Operation knotenlos flexibel beladen werden kann. Es ist besonders vorteilhaft, dass das Knochenankerelement und die Fadenführung wahlweise und/oder flexibel interoperativ anpassbar und/oder einsetzbar sind. Folglich ist das Knochenankerelement vielseitig einsetzbar und es müssen nicht zwei oder mehrere Konzepte zur Verwendung und Beladung sowie zum Setzen der Naht unter der Operation vorab ausgearbeitet werden.

Zudem ist eine Vorbeladung des Knochenankerelementes mit einem Faden mit einer zusätzlichen flexiblen Beladung mit einem Faden oder weiteren Fäden unter der Operation kombinierbar, sodass zwei oder mehrere Fäden mit dem Knochenankerelement verwendbar sind. Hierbei ist beispielsweise der erste Querdurchbruch mit einem Faden vorbeladen, während durch die distale Spaltöffnung am distalen Ende des Knochenankerelementes ein Faden interoperativ frei von einer Einfädelhilfe aufgenommen und durch Einbringen des Knochenankerelementes in den Knochen verblockt werden kann. Durch die mittige Spaltöffnung am distalen Ende ist der Faden geführt durch den zweiten Querdurchbruch von zwei sich an der Spaltöffnung gegenüberstehenden distalen Spitzenflügeln umgeben, welche ein Wiederrausrutschen des Fadens aus der Spaltöffnung verhindern.

Somit ist in einer knotenfreien Operationstechnik der Faden durch mindestens einen der Querdurchbrüche des Knochenankerelementes hindurch fädelbar, nachdem eines der freien Fadenenden unter Verwendung eines Nadel-Systems durch das abgelöste Gewebe hindurchgestochen wurde. Danach wird dieses Fadenende beim Einbringen des Knochenankerelementes zwischen dem Knochen und dem Knochenankerelement blockiert.

Ein wesentlicher Gedanke der Erfindung beruht insbesondere darauf, ein Knochenankerelement bereitzustellen, welches an seinem distalen Abschnitt in Längsrichtung zwei hintereinander angeordnete Querdurchbrüche zum Aufnehmen eines Fadens oder mehrerer Fäden aufweist, wobei jeder der beiden Querdurchbrüche jeweils flexibel manuell oder mit einer Einfädelhilfe vorbeladen und/oder unter der Operation beladen werden kann. Hierbei wirkt der Steg im plattenförmigen distalen Abschnitt zwischen den beiden Querdurchbrüchen insbesondere als Zugwiderstand und Halt für die beiden zum proximalen Ende geführten Fadenenden und/oder aufgrund seiner schrägen Anschlagsfläche als gerichtete Führung für eine Öse oder einen Faden. Somit kann der Faden als Schlaufe um den Steg herumgeführt sein, wobei entweder beide Fadenenden zum proximalen Ende des Knochenankerelementes durch den durchgehenden Hohlraum des proximalen Abschnittes, nur ein Fadenende durch den durchgehenden Hohlraum und das andere Ende außen entlang der Längsrichtung des proximalen Abschnittes zum proximalen Ende oder beide Fadenenden außen entlang der Längsrichtung des proximalen Abschnittes zum proximalen Ende geführt sind.

Somit erlaubt das Knochenankerelement ein konventionelles einschraubbares Knochenanker-, ein einschlagbares Knochenanker- und ein knotenloses Knochenankerkonzept. Dadurch ist der Knochenanker nicht nur für das Fixieren einer Sehne im Schulter- und Hüftbereich, sondern für eine Vielzahl von Operationen zum Fixieren von Sehnen und Bändern sowie von Gelenklippen, Rotatorenmanschettenruptur oder gerissenen Kapseln einsetzbar. Der Knochenanker kann beispielsweise einen maximalen Außendurchmesser von 2,8 mm, 4,0 mm oder 5,5 mm und eine jeweilige Länge von 8 mm, 15 mm oder 18 mm aufweisen. Das Verhältnis der Länge des distalen Abschnittes zu der Länge des proximalen Abschnittes beträgt dabei insbesondere 0,54, 0,33 oder 0,29.

Durch die Ausgestaltung des Stegs wird beispielsweise eine Öse eines Einfädlers, welche durch die seitliche proximale Öffnung des proximalen Abschnittes, den durchgehenden Hohlraum und die distale Öffnung längs in den ersten Querdurchbruch geschoben wird, aufgrund der schrägen Anschlagsfläche immer auf die durch die Anschlagsfläche vordefinierte Seite des distalen Abschnittes nach außen, insbesondere schräg nach außen, auf eine Seite der Plattenebene des distalen Abschnittes geführt. Somit besteht die Anwendungssicherheit, dass die Öse immer auf derselben, vordefinierten Seite des plattenförmigen distalen Abschnittes des Knochenankers nach außen austritt. Zudem wird dadurch verhindert, dass die Öse und/oder Einführungshilfe längs ausgerichtet im ersten Querdurchbruch hängen bleibt.

Bevorzugt ist die Anschlagsfläche hier nicht über die gesamte Höhe des Querdurchbruches quer zur Längsrichtung des Knochenankerelementes ausgeführt, sondern beginnt beispielsweise erst in der Mitte der Höhe des Querdurchbruches und ist schräg nach oben geführt. Hierzu weist die schräge Anschlagsfläche beispielsweise einen Winkel von 5° bis 25°, bevorzugt von 10° bis 15° zur Querachse durch den ersten Durchbruch auf, welche senkrecht zur Längsachse des Knochenankerelementes steht. Hierbei kann die schräge Anschlagsfläche nicht nur auf der proximalen Seite des Steges zum ersten Querdurchbruch ausgeführt sein, sondern auch in proximaler Richtung an den Innenseiten des distalen Abschnittes in Längsrichtung um den ersten Querdurchbruch herum ausgeführt sein. Insbesondere kann sich die in den Seitenwänden des proximalen Querdurchbruches eingebrachte schräge Anschlagsfläche in Längsrichtung auf das proximale Ende des Knochenankers rund umlaufend bis vor das proximale Ende des ersten Querdurchbruches hinziehen.

Vorteilhaft ermöglichen die Spaltöffnung am distalen Ende des zweiten Querdurchbruchs und die schräge Anschlagsfläche vor allem auch eine nachträgliche Beladung. Somit ist ein Nahtmaterial oder sind mehrere Nahtmaterialien flexibel je nach Bedarf an unterschiedlichen Stellen des Knochenankerelementes ein- und durchführbar, wobei das Beladen mit dem Nahtmaterial sowohl manuell als auch mittels eines Einbringers und/oder eines Einfädlers flexibel bei der Vorkonfektionierung und/oder bei der Anwendung des Knochenankerelementes erfolgen kann.

Folgendes Begriffliche sei erläutert:
Ein "Knochenankerelement" (auch "Knochenanker", "Knochenschraube", "Fadenanker" oder "Interferenzschraube" genannt) ist insbesondere ein Element zum Einbringen in einen Knochen und/oder Fixieren von Gewebe, insbesondere abgelöstes Gewebe, an dem Knochen. Somit ist ein Knochenanker ein Implantat zur anatomischen Fixation. Der Knochenanker weist insbesondere zwei Querdurchbrüche zum Durchfädeln eines Nahtfadens oder mehrerer Nahtfäden auf.

Unter "proximal" wird jeweils das Ende eines Gegenstandes, wie dem Knochenankerelement oder eines Einbringers, verstanden, welches in der Richtung zum Körper des Verwenders und/oder Operateurs liegt. Entsprechend wird als jeweiliges "distales Ende" oder als "distaler Abschnitt" das Ende oder der Abschnitt verstanden, welcher auf der Seite des Gegenstandes entfernt von dem Bediener des Gegenstandes und folglich auf der Seite des zu operierenden Knochens und/oder Körpers liegt. Somit wird das distale Ende des Knochenankerelementes entfernt vom Operateur in den Knochen eingebracht.

Unter "in Längsrichtung" wird die Richtung der längsten Ausdehnung und/oder Abmessung des Knochenankerelementes verstanden. Somit ist die Längsrichtung insbesondere auch die Richtung, in welcher das Knochenankerelement in einen Knochen eingeführt wird.

Der "proximale Abschnitt" (auch als "proximaler Körper" bezeichnet) weist insbesondere einen Grundkörper auf, wobei der Grundkörper in Längsrichtung als Hohlkörper und bevorzugt als Hohlzylinder mit einem runden Querschnitt ausgebildet ist. Selbstverständlich kann der proximale Abschnitt auch einen ovalen, drei- oder mehreckigen Querschnitt aufweisen. Der proximale Abschnitt weist an seinem proximalen Ende insbesondere eine proximale Öffnung auf, welche im Inneren an den durchgehender Hohlraum angrenzt, welcher bis zu einer distalen Öffnung am distalen Ende des proximalen Abschnittes durchgehend ausgebildet ist. An der distalen Öffnung des proximalen Abschnittes geht der proximale Abschnitt in den distalen Abschnitt über. Der durchgehende Hohlraum des proximalen Abschnittes kann beispielsweise als eine axiale durchgehende Bohrung ausgestaltet sein. Diese Bohrung muss aber nicht zwangsläufig einen runden Querschnitt aufweisen, sondern kann auch eine mehreckige, wie beispielsweise eine sechseckige, Form im Querschnitt aufweisen. Durch den durchgehenden Hohlraum des proximalen Abschnittes ist beispielsweise ein Nahtmaterial durchfädelbar und/oder ein Einbringer einbringbar.

Der proximale Abschnitt weist insbesondere an seiner Außenseite einen profilierten Vorsprung oder mehrere profilierte Vorsprünge auf. Bei einem "profilierte Vorsprung" handelt es sich beispielsweise um eine Rippe oder mehrere Rippen. Die Rippe oder die Rippen können einseitig, umlaufend und/oder versetzt angeordnet sein. Der profilierte Vorsprung kann auch als Gewinde ausgestaltet sein. Hierbei beginnt bevorzugt ein durchgehender Gewindegang am proximalen Ende des proximalen Abschnittes außen mit einem flachen Gewindeabschnitt und endet am distalen Ende des proximalen Abschnittes ebenfalls mit einem flachen Gewindeabschnitt bündig. Je nach Ausgestaltung des profilierten Vorsprunges ist das Knochenankerelement durch Eindrehen und/oder Einschlagen in den Knochen verankerbar.

Am distalen Ende des proximalen Abschnittes geht der proximale Abschnitt in den distalen Abschnitt über, wobei der distale Abschnitt (auch "distale Spitze" genannt) plattenförmig abgeflacht mit einer Plattenebene ausgebildet ist und somit quer zur Plattenebene eine deutlich geringere Höhe als der proximale Abschnitt aufweist.

In Längsrichtung nachfolgend der distalen Öffnung des proximalen Abschnittes schließt sich direkt der erste Durchbruch durch die Plattenebene des distalen Abschnittes an. Anschließend folgt in Längsrichtung ein Steg und dann der zweite Querdurchbruch mit einer Spaltöffnung am distalen Ende des distalen Abschnittes und somit am distalen Ende des Knochenankerelementes.

Unter einem "ersten Querdurchbruch" und einem "zweiten Querdurchbruch" (auch "Beladungsloch" genannt) wird insbesondere eine durchgehende Öffnung und somit ein Loch quer zur Plattenebene des distalen Abschnittes verstanden.

Unter einer Spaltöffnung "im Wesentlichen quer zur Plattenebene" wird insbesondere verstanden, dass die Spaltöffnung vollständig durch die Plattenebene durchgeht. Sodass ein Faden durch die Spaltöffnung, insbesondere vertikal ausgerichtet, in den zweiten Querdurchbruch hinein einfädelbar ist. Hierbei muss die Spaltöffnung jedoch nicht zwingend senkrecht zur Plattenebene stehen, sondern kann auch in einem schrägen Winkel zur Plattenebene ausgeführt sein. Somit kann die Spaltöffnung auch schräg durch die Plattenebene gehen.

Unter "Öse" wird insbesondere eine Draht- und/oder Metallschlinge verstanden. Bei einer Öse kann es sich insbesondere auch um einen zu einer Schlaufe gebogenen Beladungsdraht, aber auch um einen zu einer Schlaufe gebogenen Nahtfaden handeln. Die Öse ist insbesondere als Schlinge am Ende eines Drahtes oder Nahtfadens ausgeführt, wobei die beiden Drahtenden oder Nahtfadenenden durch einen Einfädler zusammengehalten werden, an diesem befestigt sind oder andersartig, beispielsweise manuell, zusammengehalten werden.

Unter "nach außen geführt" wird insbesondere verstanden, dass die Öse beim Auftreffen auf die schräge Anschlagsfläche durch den ersten Querdurchbruch aus der Plattenebene herausgeführt wird.

In einer weiteren Ausführungsform des Knochenankerelementes verjüngt sich der proximale Abschnitt und/oder der distale Abschnitt stetig oder unstetig vom jeweiligen proximalen Ende zum jeweiligen distalen Ende.

Dadurch wird das Einbringen des Knochenankerelementes durch ein Bohrloch in den Knochen vereinfacht und aufgrund der sich verjüngenden Form des distalen Abschnittes und/oder des proximalen Abschnittes wird das Knochenankerelement beim Einbringen innerhalb des Bohrloches optimal ausgerichtet und geführt. Zudem ist dadurch das distale Ende und/oder der distale Abschnitt des Knochenankerelementes mechanisch stabil ausgeführt und hält den Belastungen beim Einbringen stand.

Bei einem "Bohrloch" kann es sich prinzipiell auch um ein Einschlagloch oder um ein andersartig in den Knochen eingebrachtes Loch handeln.

Unter "verjüngt" wird verstanden, dass sich der Durchmesser des proximalen Abschnittes und/oder des distalen Abschnittes von ihrem jeweiligen proximalen Ende in Längsrichtung zum jeweiligen distalen Ende verkleinert. Hierbei kann die Verjüngung sowohl in Längsrichtung als auch in Querrichtung des proximalen Abschnittes und/oder des distalen Abschnittes stetig oder unstetig und somit gleichförmig oder ungleichförmig ausgebildet sein.

Um die mechanische Stabilität des Knochenankerelementes zu verbessern, ist der erste Querdurchbruch durch die Plattenebene im Wesentlichen viereckig, insbesondere quer zur Längsrichtung gesehen rechteckig, ausgebildet.

Somit weist sowohl der erste Querdurchbruch als auch der sich in Längsrichtung zum distalen Ende anschließende Steg eine ausreichende mechanische Stabilität gegenüber den Kräften beim Eintreiben des Knochenankerelementes auf. Somit kann das Knochenankerelement mit dem benötigten Druck eingebracht werden.

Zudem wird dadurch für einem Einbringer zum Einbringen des Knochenankerelementes in die Bohrung im Knochen genügend Auflage- und Kraftübertragungsfläche am Knochenankerelement bereitgestellt.

Der erste Querdurchbruch ist insbesondere gezielt frei von einem runden Querschnitt, da bei einem runden Querschnitt die Wandung des Steges zwischen dem ersten Querdurchbruch und dem zweiten Querdurchbruch mit einer zu geringen Materialstärke ausgeführt wäre, da aus anatomischen Gründen die Länge des Knochenankerelementes in Längsrichtung möglichst minimal gehalten sein sollte. Durch die viereckige Ausführung des ersten Durchbruches bietet der Steg mit einer entsprechenden Materialstärke auch einen ausreichenden Widerstand gegen die Zugkräfte des Fadens oder der Fäden.

Zudem wird durch die viereckige Form des ersten Querdurchbruches sichergestellt, dass bei Ausgestaltung des profilierten Vorsprungs als Gewindegang der letzte Gewindegang beim Übergang vom distalen Ende des proximalen Abschnittes zu dem distalen Abschnitt mit dem sich direkt nach der distalen Öffnung anschließenden ersten Querdurchbruch nicht beschädigt wird.

Um das distale Ende des distalen Abschnittes als zwei sich gegenüberstehende Klemmflügel (Clip) mit einer Klemmspannung an der Spaltöffnung auszuführen, weist der zweite Durchbruch durch die Plattenebene im Wesentlichen eine runde Form auf.

Dadurch kann schonend ein Faden durch die Spaltöffnung des zweiten Querdurchbruches eingebracht werden, wobei aufgrund der durch den zweiten runden Querdurchbruch bedingten Form des verbleibenden plattenförmigen Materials am distalen Ende des distalen Abschnittes um die Spaltöffnung und somit der Ausbildung eines Clips, das Fadenmaterial jedoch in dem zweiten Querdurchbruch durch Zusammendrücken des Spaltöffnung durch die Klemmflügel festgehalten wird. Dadurch kann durch die Spaltöffnung in einfacher Weise während einer Operation ein Faden intraoperativ ohne Einfädelhilfe in den zweiten Querdurchbruch aufgenommen und anschließend festgehalten werden, sodass ein nachträgliches Rausrutschen des Nahtfadens durch die Spaltöffnung nach außen verhindert ist.

Unter "im Wesentlichen viereckig" bezüglich des ersten Querdurchbruches und unter "im Wesentlichen eine runde Form" des zweiten Querdurchbruches wird jeweils verstanden, dass die Grundform, quer zur Plattenebene gesehen, die beanspruchte Form aufweist. Somit kann der erste Querdurchbruch bei einer im Wesentlichen viereckigen Form beispielsweise dennoch abgerundete Ecken aufweisen, um eine Abnutzung oder ein Abscheuern des Fadens zu verhindern. Ebenso muss die im Wesentlichen runde Form des zweiten Querdurchbruches nicht zwingend kreisrund sein, sondern kann beispielsweise auch oval ausgestaltet sein. Insbesondere bei den oben beispielhaft genannten Knochenankerelementen mit einem Außendurchmesser von 2,8 mm kann der erste Querdurchbruch beispielsweise eine lichte Weite von 0,8 mm (in Längsrichtung) x 1,0 mm (in Querrichtung) und der zweite Querdurchbruch eine lichte Weite von 0,9 mm haben. Im Falle eines Außendurchmessers von 4,0 mm weisen beispielsweise der erste Querdurchbruch 1,0 mm x 1,6 mm und der zweite Querdurchbruch 1,4 mm sowie bei einem Außendurchmesser von 5,5 mm der erste Querdurchbruch 1,1 mm x 1,8 mm und der zweite Querdurchbruch 1,4 mm auf.

In einer weiteren Ausführungsform des Knochenankerelementes ist das distale Ende des distalen Abschnittes um die Spaltöffnung in der Plattenebene abgerundet und/oder weist nach der Spaltöffnung einen sich erweiternden Freiraum in Längsrichtung bis zum distalen Ende auf.

Dadurch wird ein von außen in die Spaltöffnung einzubringender Faden durch die Abrundung schonend ohne Abnutzung eingefädelt und aufgrund des sich nach der Spaltöffnung erweiternden Freiraums in Längsrichtung bis zum distalen Ende und somit umgekehrt geschaut bei Richtung zum proximalen Ende ein sich verengender Freiraum bis zur Spaltöffnung ein Einfädeln des Fadens durch die Spaltöffnung in den zweiten Querdurchbruch hinein erleichtert. Beispielsweise können die beiden Clip-Flügel um die Spaltöffnung herum konvex abgerundet sein.

Somit weist der distale Abschnitt insbesondere auch zwei abgerundete distale äußere Ecken auf, welche sowohl ein fadenschonendes Einfädeln als auch ein erleichtertes Einbringen des Knochenankerelementes in den Knochen ermöglichen.

In einer weiteren Ausführungsform des Knochenankerelementes ist eine Spaltbreite der Spaltöffnung in der Plattenebene an einen Durchmesser des Nahtmaterials angepasst.

Hierbei kann die Spaltbreite gezielt nur auf den Durchmesser eines Nahtmaterials angepasst sein oder aufgrund des Zusammenwirkens der Spaltbreite mit den umgebenden Clip-Flügeln des distalen Endes des distalen Abschnittes kann die Spaltbreite entsprechend eine Spannung auf die Clip-Flügel induzieren, sodass beispielsweise Nahtmaterial mit verschiedenen Durchmessern durch die Spaltöffnung einfädelbar und im zweiten Querdurchbruch haltbar ist. Hierbei wird dann entsprechend das jeweilige Nahtmaterial je nach seinem Durchmesser mit einem entsprechend unterschiedlichen Kraftaufwand durch die Spaltbreite der Spaltöffnung eingefädelt (eingeclipt).

Insbesondere ist die Spaltbreite der Spaltöffnung derart angepasst, dass Nahtmaterial der Fadenstärke USP 2 in den zweiten Querdurchbruch einfädelbar ist. Hierfür kann die Spaltbreite beispielsweise 0,4 mm betragen.

Unter einer "Spaltbreite" wird insbesondere die Abmessung der Spaltöffnung quer zur Längsrichtung des Knochenankerelementes verstanden.

In einer weiteren Ausführungsform ist das Knochenankerelement einstückig ausgebildet.

Somit muss das Knochenankerelement unter der Operation nicht aus mehreren Teilen zusammengesetzt oder vom Operateur modifiziert werden. Zudem ist das Knochenankerelement in einfacher Weise durch Spritzguss fertigbar. Es ist besonders vorteilhaft, dass mit einem einstückigen, einzigen Knochenankerelement eine Vielzahl von Beladungsmöglichkeiten mit einem Faden und eine Vielzahl entsprechender Operationstechniken durchführbar sind.

Damit das Knochenankerelement im Knochen und somit im menschlichen Körper verbleiben kann, weist das Knochenankerelement ein biointegrierbares Material auf.

Somit kann sich das Knochenankerelement mit der Zeit nach Anwachsen des abgelösten Gewebeteils im Körper auflösen, wird vom Körper abgebaut und/oder verbleibt bioverträglich im Körper. Bevorzugt wird das biointegrierbare Material langfristig vollständig durch Knochenmaterial ersetzt. Zudem wird aufgrund der Verwendung eines solchen Materials eine Schädigung des umgebenden Gewebes und/oder des Knochens sowie eine Störung von bildgebenden Diagnostikverfahren vermieden.

Bei einem "biointegrierbaren Material" handelt es sich insbesondere um ein biologisch verträgliches Material. Bei dem biointegrierbaren Material kann es sich insbesondere um ein bioresorbierbares, biokompatibles und/oder ein biologisch abbaubares Material, beispielsweise ein abbaubares Polymer und/oder einen Kunststoff, handeln. Beispielsweise kann das biointegrierbare Material Tricalciumphosphat aufweisen. PEEK ist hingegen nicht abbaubar und weist jedoch hier insbesondere die Vorteile der Akzeptanz für chirurgische Anwendungen, der mechanischen Stabilität, der Sterilisierbarkeit und der guten Herstellbarkeit auf. Ebenso kann es sich bei dem biointegrierbaren Material um ein Metall und/oder eine Metalllegierung handeln.

Um ein Einwachsen des Knochens in das Knochenankerelement und somit eine Einheilung im Knochen zu beschleunigen, kann der proximale Abschnitt ein Perforationsloch oder mehrere Perforationslöcher aufweisen.

Somit hat der als Hohlkörper ausgeführte proximale Abschnitt eine oder mehrere Durchbohrungen. Bevorzugt sind mehrere Perforationslöcher zueinander versetzt rundumlaufend entlang der Längsrichtung ausgebildet.

Um einen Einbringer (auch Eintreiber genannt) mit dem Knochenankerelement zum Einbringen des Knochenankerelementes in das Bohrloch im Knochen zu verbinden, weist die proximale Öffnung ein Innenmitnahmeprofil, insbesondere ein Innensechskantprofil, auf.

Somit kann die notwendige axiale Kraft und/oder ein Drehmoment auf den Einbringer aufgebracht und über das Innenmitnahmeprofil auf das Knochenankerelement zum Einschrauben und/oder Einschlagen in den Knochen übertragen werden.

Das Innenmitnahmeprofil ist insbesondere an der proximalen Öffnung in Richtung der Längsrichtung in der Innenwand des proximalen Abschnittes um den durchgehenden Hohlraum ausgebildet. Hierbei kann das Innenmitnahmeprofil auch im Anfangsbereich des durchgehenden Hohlraums ausgebildet sein oder auch im gesamten durchgehenden Hohlraum bis zur distalen Öffnung am distalen Ende des proximalen Abschnittes ausbildet sein, sodass die Kraftübertragung des Einbringers auf das Knochenankerelement beim Einbringen verbessert ist. Die Länge des Innenmitnahmeprofils hängt insbesondere von der Länge des Knochenankerelementes und des entsprechend verwendeten Einbringers ab.

In einer weiteren Ausführungsform des Knochenankerelementes weist das Knochenankerelement an seinem proximalen Ende und/oder an der proximalen Öffnung ein Verdrehschutzelement auf.

Somit wird nach Aufstecken und/oder Verbinden eines Einbringers an und/oder in dem Knochenankerelement ein Verdrehen des Knochenankerelementes relativ zum Einbringer beispielsweise während des Einschraubens des Knochenankerelementes in den Knochen verhindert. Zudem kann dadurch sichergestellt werden, dass das Knochenankerelement definiert ausgerichtet an dem Einbringer verbunden ist und dadurch über die schräge Anschlagsfläche des Knochenankerelementes die Öse eines Einfädlers stets nach oben oder stets auf dieselbe Seite des distalen Abschnittes über die distale Spitze des Knochenankerelementes hinausgeschoben wird.

Unter einem "Verdrehschutzelement" wird insbesondere ein konstruktives Bauelement verstanden, welches derart ausgestaltet ist, dass es ein falsches Einsetzen und/oder ungewolltes Verdrehen des mit einem Einbringer verbundenen Knochenankerelementes verhindert. Bei einem Verdrehschutzelement kann es sich beispielsweise um eine Nut handeln, in welche eine Rippe des Einbringers einrastet oder eingeklemmt wird.

In einem weiteren Aspekt der Erfindung wird die Aufgabe gelöst durch einen Einbringer zum Einbringen eines Knochenankerelementes in ein Bohrloch in einem Knochen, wobei der Einbringer einen Werkzeugschaft in einer Längsrichtung aufweist, an einem proximalen Ende des Werkzeugschaftes ein Handgriff und in einem gegenüberliegenden, distalen Endabschnitt ein Mitnehmerprofil zum Verbinden mit dem Knochenankerelement angeordnet sind und der Werkzeugschaft zumindest in einem Bereich vor dem distalen Ende bis einschließlich des Mitnehmerprofils einen durchgehenden Hohlraum in der Längsrichtung aufweist, wobei der Werkzeugschaft vor dem distalen Ende eine Einführöffnung in Längsrichtung aufweist, wobei die Einführöffnung mit dem durchgehenden Hohlraum verbunden ist, sodass von außen eine Öse durch die Einführöffnung in den durchgehenden Hohlraum bis durch das Mitnehmerprofil am distalen Ende hinausschiebbar ist.

Somit wird ein Einbringer bereitgestellt, mit welchem ein zuvor beschriebenes Knochenankerelement nach Verbinden des distalen Endes des Einbringers mit dem proximalen Abschnitt des Knochenankerelementes in ein Bohrloch innerhalb eines Knochens eingeführt und dort durch Einschlagen und/oder Eindrehen verankert werden kann.

Das Mitnehmerprofil des Einbringers wird zum Verbinden mit dem proximalen Abschnitt des Knochenankerelementes bevorzugt am proximalen Ende des Knochenankerelementes in den durchgehenden Hohlraum seines proximalen Abschnittes geschoben. Je nach Länge des jeweiligen Knochenankerelementes in Längsrichtung greift hierbei das Mitnehmerprofil des Einbringers am Ende des proximalen Abschnittes vor der distalen Öffnung axialseitig sowie proximalseitig im Bereich der proximalen Öffnung des Innenmitnahmeprofils des Knochenankerelementes ein, der Eingriff erfolgt nur am distalseitigen Ende des proximalen Abschnittes und/oder nur am proximalseitigen Ende des proximalen Abschnittes.

Bevorzugt stützt sich der Einbringer in axialer Richtung an der proximalen Seite des Knochenankerelements, an einem Absatz am distalen Ende des durchgehenden Hohlraums oder an beidem ab. Insbesondere stützt sich der Einbringer in axialer Richtung an der distalen Seite des Knochenankerelementes ab und/oder wird zudem darüber geklemmt. Dadurch kann für alle verschiedenen Größen von Knochenankerelementen ein einziger Einbringer verwendet werden.

Es ist besonders vorteilhaft, dass der Einbringer eine Einführöffnung aufweist, wodurch eine Öse eines Einfädlers intuitiv nach vorne über das distale Ende durch den Hohlraum im Werkzeugschaft des Einbringers und den durchgehenden Hohlraum des verbundenen Knochenankerelements hinausschiebbar ist. Zudem ist ein Einfädler durch die in Längsrichtung ausgeführte Einführöffnung im Werkzeugschaft geführt und gehalten, sodass dieser nicht herausfallen kann. Weiterhin ist vorteilhaft, dass der Faden hierbei in einer sterilen Umgebung durch das Innere des Knochenankerelementes und somit des Implantates selbst geführt und geschützt wird.

Prinzipiell kann ein Faden, beispielsweise gebogen zu einer Schlaufe, auch direkt ohne Einfädler durch die Einführöffnung und den durchgehenden Hohlraum des Einbringers eingefädelt und geführt werden.

Ein "Einbringer" ist insbesondere ein Werkzeug zum Einbringen eines Knochenankers in ein Bohrloch in einem Knochen. Der Einbringer kann bereits mit einem Faden vorkonfektioniert sein oder der Faden wird nach Verbinden des Knochenankerelementes mit dem Einbringer eingefädelt. Der Einbringer weist insbesondere am proximalen Ende einen Handgriff, beispielsweise aus Kunststoff, und am distalen Ende ein Mitnehmerprofil auf. Der Werkzeugschaft des Einbringers ist bevorzugt elektropoliert und passiviert. Der Werkzeugschaft ist entweder vollständig als Hohlschaft ausgebildet oder weist nur an seinem vorderen distalen Ende einen durchgehenden Hohlraum in Längsrichtung auf, wobei der Hohlraum mit der Einführöffnung verbunden ist. Die Länge des Werkzeugschaftes ist insbesondere an den Einsatzzweck anpassbar, so ist der Werkzeugschaft bei einer Hüftoperation beispielsweise um 80 mm länger als bei einer Schulteroperation. Ebenso kann der Werkzeugschaft je nach Einsatzweck einen unterschiedlichen Durchmesser aufweisen. Bevorzugt ist der Einbringer universell für verschiedene Größen von Knochenankerelementen nutzbar.

Unter "in einem Bereich vor dem distalen Ende" wird insbesondere proximal von dem distalen Ende verstanden.

Eine "Einführöffnung" (auch "Einführfenster" oder "Schlitz" genannt) ist insbesondere eine Aussparung in Längsrichtung des Werkzeugschaftes im Bereich vor dem distalen Ende, wobei die Aussparung von außen bis in den innengelegenen Hohlschaft des Werkzeugschafts durchgeht. Die Einführöffnung ist bevorzugt länglich in Längsrichtung des Werkzeugschafts ausgeführt. Bei einer Einführöffnung kann es sich auch um einen Schlitz oder eine bis zum Hohlraum nach Innen durchgehende Nut handeln.

In die Einführöffnung wird insbesondere von außen und oben in Richtung vom proximalen Ende des Einbringers zum distalen Ende des Einbringers eine Öse eines Einfädlers oder eine Drahtschlinge eingebracht. Dadurch, dass die Öse durch den ersten Querdurchbruch des Knochenankerelementes nach außen über die distale Spitze des Knochenankerelementes hinausgeschoben werden kann, kann durch die Öse ein Faden eingefädelt werden und anschließend der Faden durch die Spaltöffnung in den zweiten Querdurchbruch gegen die distale Seite des Steges gezogen werden. Nachfolgend wird ein Fadenende außerhalb des Knochenankers bis zu seinem proximalen Ende geführt, während das andere Ende mithilfe der Öse durch Rückziehen des Einfädlers durch die distale Öffnung des durchgehenden Hohlraums zurück bis über die proximale Öffnung des proximalen Abschnittes des Knochenankers herausgezogen wird. Somit ermöglicht der Einbringer eine knotenlose Beladung des Knochenankers mithilfe eines Einfädlers oder eines Beladungsdrahtes.

In einer weiteren Ausführungsform des Einbringers weist das Mitnehmerprofil ein Außenmitnehmerprofil, insbesondere ein Außensechskantprofil auf.

Dadurch kann eine optimale Verbindung und Kraftübertragung des Einbringers auf ein Innenmitnahmeprofil, insbesondere ein Innensechskantprofil, des Knochenankerelementes erfolgen. Hierbei sind das Innenmitnahmeprofil des Knochenankerelementes und das Außenmitnehmerprofil des Einbringers entsprechend korrespondierend, zusammenwirkend ausgebildet. Folglich stellt der Einbringer einen Hexalantrieb für das Knochenankerelement dar.

Um eine definierte Ausrichtung des Knochenankerelementes am distalen Ende des Einbringers zu gewährleisten und ein Verdrehen des verbundenen Knochenankerelementes zu verhindern, weist der Einbringer insbesondere ein Verdrehschutzelement auf, sodass im Falle des Verbindens des Einbringers mittels des Mitnehmerprofils mit einem Knochenankerelement ein Verdrehen des verbundenen Knochenankerelementes verhindert ist.

Dadurch kann sichergestellt werden, dass sowohl die Einführöffnung im Werkzeugschaft des Einbringers als auch der Austritt der Öse durch den ersten Querdurchbruch nach oben jeweils an der Oberseite des Einbringers und des Knochenankers liegen.

Bei einem "Verdrehschutzelement" handelt es sich prinzipiell um ein bereits oben definiertes Verdrehschutzelement. Bevorzugt weist dazu sowohl der Einbringer als auch das proximale Ende des Knochenankerelementes jeweils ein Bauteil auf, welche zum Ausbilden des Verdrehschutzes ineinandergreifen. Beispielsweise sind die beiden korrespondierenden Verdrehschutzelemente am Einbringer und am Knochenankerelement als Stecker und Buchse oder als Rastelemente ausgebildet.

In einer weiteren Ausführungsform des Einbringers weist der Handgriff eine Aussparung und/oder eine Spule zur Aufnahme eines Nahtmaterials auf.

Somit kann der Einbringer beladen mit einem Nahtmaterial bereits vorkonfektioniert bereitgestellt werden. Ebenso können nach dem Beladen eines Knochenankerelementes mit einem Faden die beiden Fadenenden in der Aussparung des Handgriffes des Einbringers aufgenommen und/oder auf dessen Spule aufgewickelt werden. Bevorzugt werden die Fadenenden seitlich am Griff in Längsrichtung zum proximalen Ende bündig und eng anliegend geführt. Selbstverständlich kann der Handgriff auch mehrere Aussparungen in Längsrichtung aufweisen, sodass in jeder Aussprung lediglich ein Faden geführt wird. Die Spule zum Aufwickeln von überstehendem Fadenmaterial ist bevorzugt am proximalen Ende des Handgriffes ausgebildet.

Bei einer "Aussparung" handelt es sich insbesondere um eine Nut, welche in die äußere Oberfläche des Handgriffes vom proximalen bis zum distalen Ende des Handgriffes in Längsrichtung ausgeführt ist.

In einem weiteren Aspekt der Erfindung wird die Aufgabe gelöst durch ein Knochenankersystem mit einem zuvor beschriebenen Knochenankerelement und einem zuvor beschriebenen Einbringer, sodass der Einbringer mit dem Knochenankerelement verbindbar, ein Nahtmaterial durch das Knochenankerelement flexibel einfädelbar und das Knochenankerelement mittels des Einbringers in ein Bohrloch in einem Knochen einbringbar ist.

Folglich wird ein vorkonfektioniertes Knochenankersystem mit zueinander korrespondierend und zusammenwirkend ausgebildetem Knochenankerelement und Einbringer bereitgestellt.

In einer weiteren Ausführungsform des Knochenankersystems ist dem Knochenankersystem ein Einfädler mit einer Öse zugeordnet oder das Knochenankersystem weist den Einfädler mit der Öse auf, sodass im Falle des Verbindens des Einbringers mit dem Knochenankerelement die Öse des Einfädlers von außen durch die Einführöffnung in und durch den Hohlraum des Werkzeugschaftes des Einbringers durch den durchgehenden Hohlraum, die distale Öffnung und den ersten Querdurchbruch über die schräge Anschlagsfläche bis nach außen über das distale Ende des Knochenankers schiebbar und in die Öse ein Nahtmaterial einfädelbar ist.

Um ein vollständig vorkonfektioniertes Knochenankersystem bereitzustellen, weist das Knochenankersystem das Nahtmaterial auf.

In einem zusätzlichen Aspekt der Erfindung wird die Aufgabe gelöst durch ein Verfahren zum Konfektionieren eines Knochenankersystems mittels eines zuvor beschriebenen Knochenankerelementes und einem zuvor beschriebenen Einbringer oder mittels eines zuvor beschriebenen Knochenankersystems, mit folgenden Schritten:
- Verbinden des distalen Endes des Einbringers mit dem proximalen Ende des Knochenankerelementes,
- Einführen einer Öse eines Einfädlers von außen durch die Einführöffnung in den Hohlraum des Werkzeugschaftes des Einbringers,
- Durchschieben der Öse durch den Hohlraum des Werkzeugschaftes des Einbringers und durch den durchgehenden Hohlraum, die distale Öffnung und den ersten Querdurchbruch über die schräge Anschlagsfläche bis nach außen über das distale Ende des Knochenankerelementes,
- Einfädeln eines Nahtfadens des Nahtmaterials durch die Öse,
- Teilweises Zurückziehen des Einfädlers, sodass die Öse in Richtung auf das proximale Ende des Knochenankerelementes zur Spaltöffnung des zweiten Querdurchbruches verschoben wird,
- Einfädeln des Nahtfadens in die Spaltöffnung des zweiten Querdurchbruches,
- Weiteres Zurückziehen des Einfädlers, sodass der Nahtfaden an der distalen Seite des Steges anliegt,
- Ziehen eines ersten Endes des Nahtfadens aus der Öse und Führen des ersten Endes entlang einer Außenseite des Knochenankerelementes bis über sein proximales Ende,
- vollständiges Zurückziehen des Einfädlers aus der Einführöffnung des Werkzeugschaftes heraus, sodass ein zweites Ende des Nahtfadens über den Steg und durch den durchgehenden Hohlraum des proximalen Abschnittes bis über das proximale Ende des Knochenankerelementes aus der Einführöffnung herausgezogen wird, und
- Festhalten oder Befestigen der beiden Enden des Nahtfadens an dem Handgriff des Einbringers,
sodass der Nahtfaden knotenlos am Knochenankerelement befestigt ist.

Ergänzend kann vor einem Einbringen des Knochenankerelementes ein zweiter Nahtfaden mit den zuvor beschriebenen Schritten an dem Knochenankerelement eingefädelt werden. Ebenso kann zusätzlich manuell ein Nahtfaden durch die Spaltöffnung des zweiten Querdurchbruches eingefädelt und/oder mittels eines Einfädlers der erste Querdurchbruch beladen werden. Auch kann der Knochenanker, bevor dieser mit einem Nahtfaden oder mehreren Nahtfäden wie oben beschrieben beladen wird, bereits einen vorbeladenen Nahtfaden aufweisen, welcher um die distale Seite des Steges geführt ist und beide Fadenenden durch den durchgehenden Hohlraum bis über das proximale Ende hinaus vorgezogen sind.

Nachdem ein Knochenanker auf gleiche oder verschiedene Weise mit einem Nahtfaden oder mit mehreren Nahtfäden beladen ist, kann die Aufgabe in Ergänzung zu dem oben beschriebenen Verfahren zum Konfektionieren eines Knochenankersystems zusätzlich und separat gelöst werden durch ein Verfahren zum Einbringen eines Knochenankerelementes in einen Knochen und/oder zum Fixieren von Gewebe an dem Knochen mit folgenden zusätzlichen Schritt:
- Einbringen des Knochenankerelementes mittels des Einbringers in einem Bohrloch im Knochen, sodass das Knochenankerelement im Knochen verankert und der Nahtfaden knotenlos am Knochenankerelement befestigt ist.

Anschließend werden die Fadenenden zum Vernähen eines abgelösten Gewebes an den Knochen verwendet.

Somit wird ein Verfahren zum Konfektionieren eines Knochenankersystems mittels des Knochenankerelementes bereitgestellt, bei dem in einem vielfältigen Konzept das Knochenankerelement konventionell mit einem Faden beladenen, mithilfe eines Einfädlers beladen und/oder manuell während einer Operation (knotenlos) beladen werden kann.

Im Weiteren wird die Erfindung anhand von Ausführungsbeispielen näher erläutert. Es zeigen
- Figur 1: eine perspektivische Darstellung einer Knochenschraube in Ansicht auf ihr distales Ende,
- Figur 2: eine perspektivische Darstellung der Knochenschraube aus Figur 1 in Ansicht auf ihr proximales Ende,
- Figur 3: eine perspektivische Darstellung einer Alternative der Knochenschraube mit Perforationslöchern in Seitenansicht,
- Figur 4: einen Längsschnitt durch die Alternative der Knochenschraube aus Figur 3,
- Figur 5: einen Längsschnitt durch die Alternative der Knochenschraube aus Figuren 3 und 4 mit eingebrachtem Hohlschaft eines Einbringers,
- Figur 6: eine Darstellung einer konventionell mit einem Faden beladenen Knochenschraube,
- Figur 7: eine Darstellung einer mithilfe eines Einfädlers knotenlos beladenen Knochenschraube,
- Figur 8: eine Darstellung einer manuell während einer Operation beladenen Knochenschraube,
- Figur 9: eine perspektivische Darstellung des distalen Endes einer Knochenschraube mit einer schrägen Anschlagsfläche eines ersten Beladungsloches,
- Figur 10: eine perspektivische Darstellung eines Einbringers mit verbundener Knochenschraube und eingesetzten Einfädler,
- Figur 11: eine teils perspektivische Darstellung und teils Schnittdarstellung als Ausschnitt der Figur 10,
- Figur 12: einen vergrößerten Ausschnitt der Knochenschraube aus Figur 11 mit eingeschobener Öse,
- Figur 13: eine perspektivische Darstellung eines distalen Endes eines Einbringers mit verbundener Knochenschraube, eingesetzten Einfädler und Faden,
- Figur 14: eine perspektivische Darstellung eines distalen Endes eines Einbringers mit verbundener Knochenschraube und eingesetzten Einfädler mit Faden an einem ersten Beladungsloch.

Eine Knochenschraube 101 weist ein proximales Ende 103 und ein distales Ende 105 auf. In einer Längsrichtung 113 der Knochenschraube 101 schließt sich am proximalen Ende 103 ein proximaler Körper 107 an, welcher innenliegend eine Längsbohrung 117 aufweist. Außenliegend ist entlang des proximalen Körpers 107 ein durchgehendes Gewinde 115 beginnend vom proximalen Ende 103 bis zu einer distalen Öffnung 121 am distalen Ende der Längsbohrung 117 geführt.

Im Bereich der distalen Öffnung 121 geht der proximale Körper 107 in eine distale Spitze 109 mit einer Plattenebene 111 über. In der Längsrichtung 113 schließt sich an die distale Öffnung 121 ein erstes Beladungsloch 123 an, welches quer durch die Plattenebene 111 geführt ist. Das erste Beladungsloch 123 weist eine rechteckige Form quer zur Längsrichtung 113 auf, wobei die Ecken der rechteckigen Form abgerundet sind. An das erste Beladungsloch 123 schließt sich in der Längsrichtung 113 ein Steg 135 an. Weiter in der Längsrichtung 113 folgt dem Steg 135 ein zweites Beladungsloch 125, welches als runder Durchbruch durch die Plattenebene 111 ausgeführt ist und an seinem distalen Ende eine Spaltöffnung 129 aufweist.

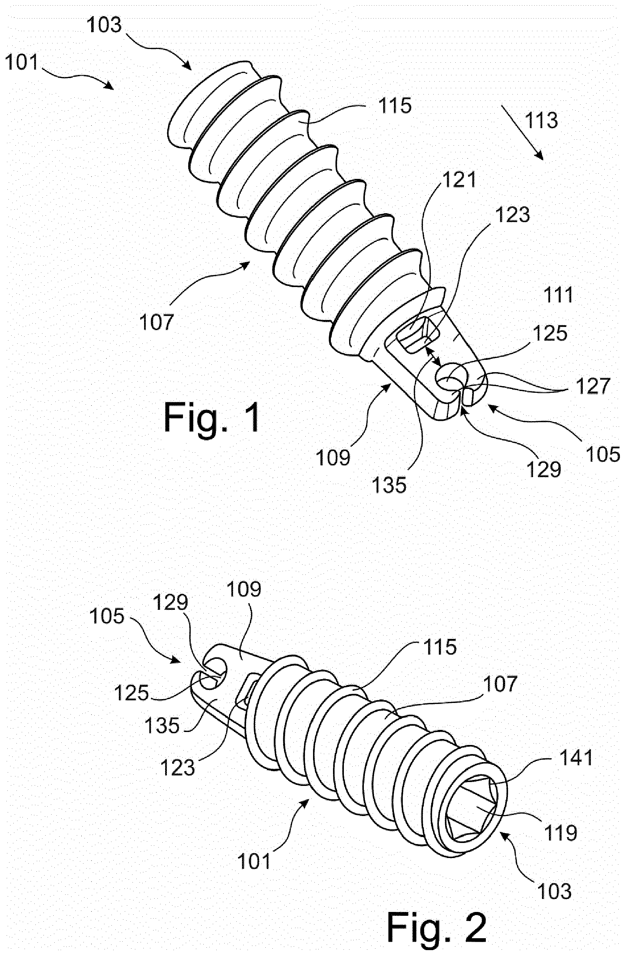

Die distale Spitze 109 verjüngt sich von der distalen Öffnung 121 bis zum distalen Ende 105 der Knochenschraube 101 (siehe Figur 1). Aufgrund dieser Verjüngung, der runden Form des zweiten Beladungsloches 125 und der Spaltöffnung 129 sind beidseitig des zweiten Beladungsloches 125 jeweils ein Klemmflügel 127 ausgebildet.

Am proximalen Ende 103 ist innenliegend in der proximalen Öffnung 119 der durchgehenden Längsbohrung 117 ein Innensechskantprofil 141 angeordnet (Fig. 2).

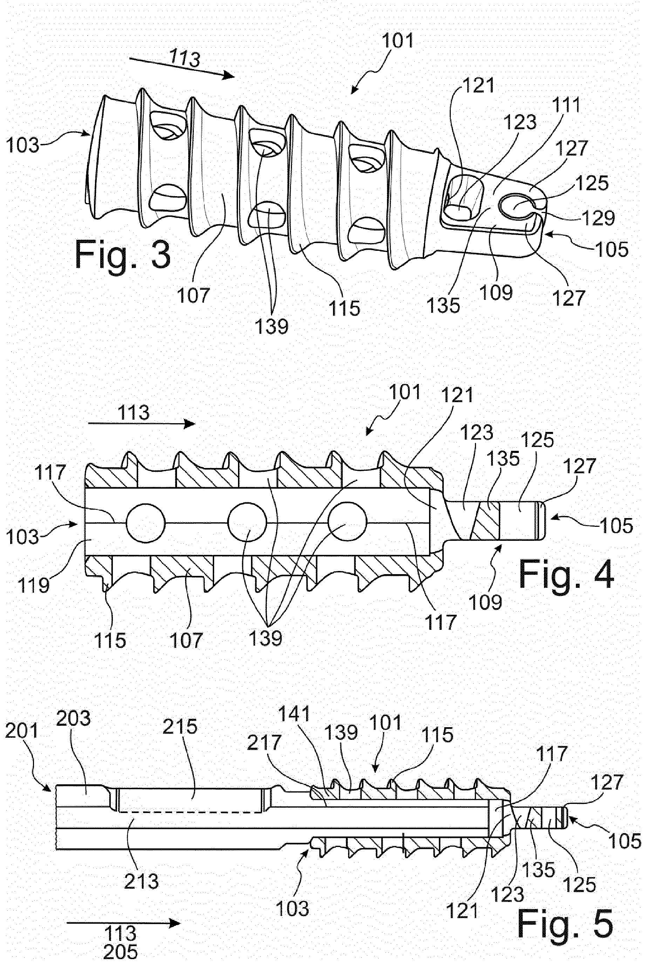

In einer Alternative der Knochenschraube 101 (Figur 3 und Figur 4) weist die Knochenschraube 101 zusätzlich durchgehend durch den proximalen Körper 107 mehrere Perforationslöcher 139 auf, welche regelmäßig beabstandet über die Querschnittsoberfläche und in Längsrichtung 113 des proximalen Körpers 107 (und somit der Mantelfläche) verteilt sind, um ein späteres Einwachsen eines Knochens in die Knochenschraube 101 zu verbessern.

In die Längsbohrung 117 im Inneren der Knochenschraube 101 ist ein Hohlschaft 203 eines Einbringers 201 mit einem Außensechskantprofil 217 an seinem distalen Ende eingeführt. Entsprechend weist der Hohlschaft 203 eine Längsrichtung 205 auf, welche mit der Längsrichtung 113 der Knochenschraube 101 zusammenfällt. Das Außensechskantprofil 217 liegt innenliegend an den Wänden der Längsbohrung 117 der Knochenschraube 101 an. An deren proximalem Ende 103 liegt eine Schulter des Hohlschafts 203 an.

Des Weiteren weist der Hohlschaft 203 des Einbringers 201 einen Schlitz 215 auf, welcher vor dem Außensechskantprofil 217 angeordnet und mit dem Hohlraum 213 verbunden ist (siehe Figur 5).

Des Weiteren ist in dieser Alternative der Knochenschraube 101 das erste Beladungsloch 123 quer zur Längsrichtung 113 nach oben (wie in Figur 4 gezeigt) näherungsweise konisch erweitert. Mittels einer nicht gezeigten Einfädelhilfe wird eine Drahtschlinge der Einfädelhilfe von außen durch den Schlitz 215 und den Hohlraum 213 des Hohlschaftes 203 in Längsrichtung 205 zur distalen Öffnung 121 geschoben. Aufgrund der sich nach oben etwa konusförmig erweiternden Form des ersten Beladungslochs 123 quer zu den Längsrichtungen 205 und 113 wird die Drahtschlinge nach oben durch die obere Öffnung des ersten Beladungsloches 123 nach außen geleitet, sodass diese über der oberen Seite des Steges 135 liegt (nicht in Fig. 5 gezeigt). Anschließend kann ein nicht gezeigter Nahtfaden durch diese Drahtschlinge eingefädelt werden.

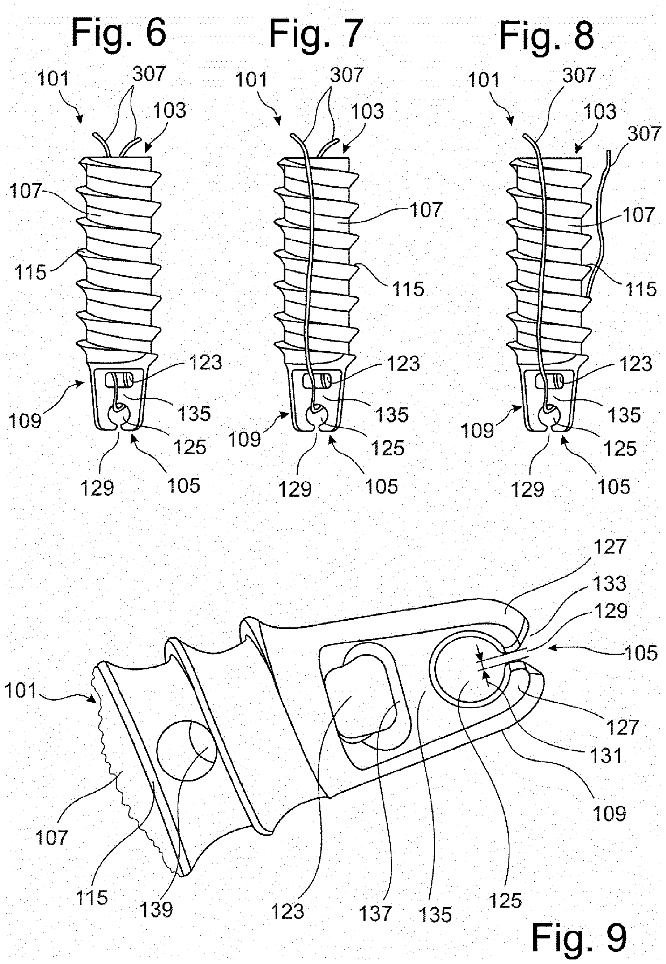

Die Knochenschraube 101 kann in unterschiedlicher Weise mit einem Faden 307 beladen werden. Bei einer konventionellen Beladung ist der Faden 307 durch die Spaltöffnung 129 um das distale Ende des Steges 135 und dessen Seitenwände herumgeführt und die beiden Fadenenden gehen durch die Längsbohrung 117 im Inneren des proximalen Körpers 107 und die proximale Öffnung 119 am proximalen Ende 107 der Knochenschraube nach außen (siehe Figur 6).

Bei einer knotenlosen Beladung der Knochenschraube 101 mithilfe eines Einfädlers ist der Faden ebenfalls durch das zweite Beladungsloch 125 um die distale Seite des Steges 135 geführt, wobei ein Fadenende außen über das Gewinde 115 bis zum proximalen Ende 103 der Knochenschraube 101 geführt und das andere Fadenende durch die Längsbohrung 117 im Inneren der Knochenschraube 101 bis zum proximalen Ende 103 hochgezogen sind (siehe Figur 7).

In einer dritten knotenlosen Beladungsmöglichkeit ist der Faden 307 manuell durch den Operateur durch die Spaltöffnung 129 in das zweite Beladungsloch 125 eingefädelt worden und liegt wiederum um das distale Ende des Steges 135 herum. Die beiden Enden des Fadens 307 sind außen über das Gewinde 117 bis zum proximalen Ende 103 hochgezogen (siehe Figur 8).

Alle drei in den Figuren 6 bis 8 dargestellten Beladungsvarianten der Knochenschraube 101 mit einem oder mehreren Fäden lassen sich beliebig untereinander kombinieren.

In einer Alternative der Knochenschraube 101 mit Perforationslöchern 139 weist das erste Beladungsloch 123 eine schräge Anschlagsfläche 137 am distalen Ende des ersten Beladungsloches 123 auf, wobei die schräge Anschlagsfläche 137 beidseitig in Richtung des proximalen Endes 103 auslaufend ausgebildet ist (siehe Fig. 9). Die schräge Anschlagsfläche 137 setzt in der Mitte einer Höhe der distalseitigen Wand um das erste Beladungsloch 123 an und weist eine Schräge von 10° oder 15° auf. Durch diese schräge Anschlagsfläche 137 wird eine durch die Längsbohrung 117 des proximalen Körpers 107 und durch die distale Öffnung 121 längs in das erste Beladungsloch 123 geschobene Öse nach oben und außen über den Steg 135 abgelenkt und geführt.

Des Weiteren ist in Figur 9 das distale Ende 105 der distalen Spitze 109 in Vergrößerung gezeigt. Die Spaltöffnung 129 weist eine Spaltbreite 131 auf. In Längsrichtung 113 zum distalen Ende 105 folgt der Spaltöffnung 129 mit der konstanten Spaltbreite 131 ein sich konisch öffnender Freiraum 133, sodass beidseitig des zweiten Beladungslochs 125 und der Spaltöffnung 129 zwei Klemmflügel 127 ausgebildet sind. Hierbei ist die Spaltbreite 131 der Spaltöffnung 129 derart gewählt, dass ein Faden unter Kraftaufwendung manuell oder mittels einer Einfädelhilfe von außen geführt durch den konischen Freiraum 133 in das zweite Beladungsloch 125 gedrückt werden kann.

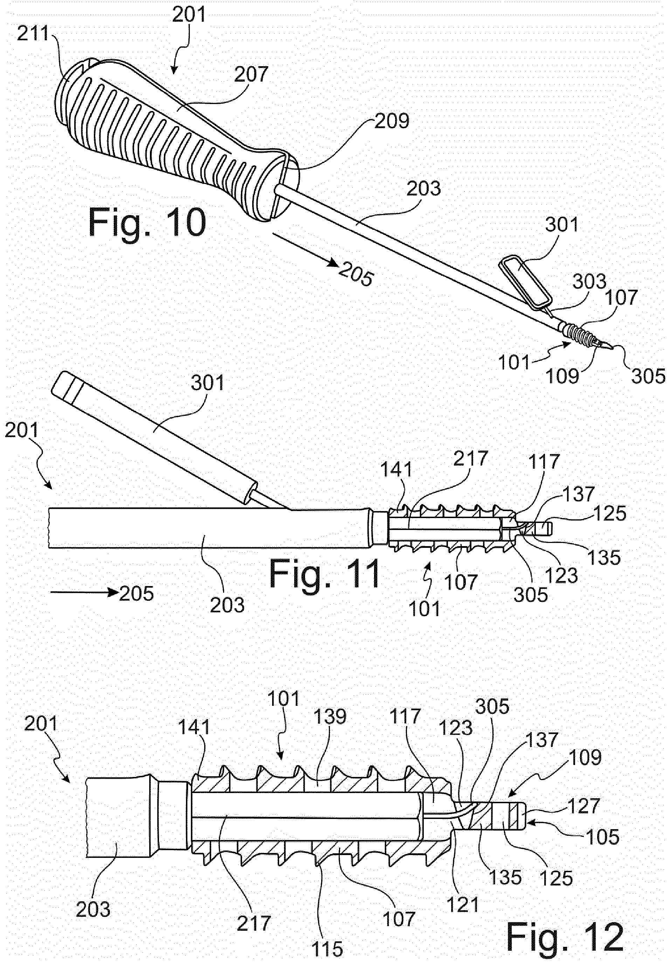

Ein Knochenankersystem besteht aus einem Einbringer 201 und einer Knochenschraube 101 (Figur 10). Der Einbringer 201 weist einen Handgriff 207 auf. Vom distalen Ende des Handgriffes 207 ist oben und unten im Handgriff jeweils eine Nut 209 bis zum proximalen Ende des Handgriffes 207 geführt. Am proximalen Ende des Handgriffes 207 ist eine Fadenspule 211 angeordnet.

Des Weiteren weist der Einbringer 201 einen Hohlschaft 203 auf, wie dieser bereits in der Figur 5 gezeigt ist. Durch den Schlitz 215 des Hohlschaftes 203 ist ein Einfädler 301 mit einem Draht 303 und einer endständigen Öse 305 eingesteckt, wobei der Draht 303 und die Öse 305 durch den Hohlraum 213 des Hohlschaftes 203 und durch die Längsbohrung 117 der Knochenschraube 101 geführt sind, sodass die Öse 305 bis über das distale Ende 105 der distalen Spitze 109 herausgeschoben ist (siehe Figur 10).

Aufgrund der schrägen Anschlagsfläche 137 des ersten Beladungsloches 123 wird die Öse 305 nach oben durch das erste Beladungsloch 123 abgelenkt (die Figuren 11 und 12 zeigen den Zustand der auf der schrägen Anschlagsfläche 137 gerade auftreffenden Öse 305).

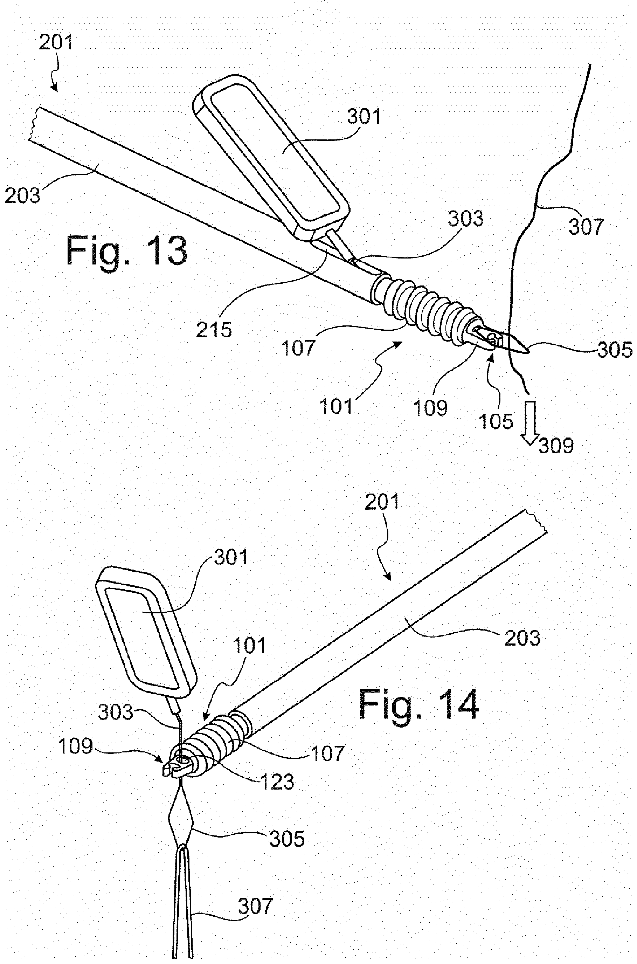

Wie insbesondere in Figur 13 gezeigt, wird dadurch die Öse 305 auf der Oberseite der distalen Spitze 109 über das distale Ende 105 hinaus durchgeschoben und ein Faden 307 kann in einer Einfädelrichtung 309 durch die Öse 305 gezogen werden. Anschließend wird der Einfädler 301 wieder aus dem Schlitz 215 so weit herausgezogen, dass die Öse 305 in den Bereich des ersten Beladungsloches 123 mit der Spaltöffnung 129 gelangt und der Faden 307 durch die Spaltöffnung 129 in das zweite Beladungsloch 125 eingefädelt wird. Daraufhin wird, wie oben zur Figur 7 beschrieben, weiter vorgegangen.

In einer Alternative wird der Draht 303 eines Einfädlers 301 mit einer am Ende angeordneten Öse 305 durch das erste Beladungsloch 123 geschoben und durch die Öse wird ein Faden 307 durchgefädelt, wobei die beiden Fadenenden entgegen einer Richtung des Einfädlers 301 ausgerichtet sind. Anschließend wird der Einfädler 301 so weit nach oben gezogen, dass die Öse 305 mit den Fadenenden durch das erste Beladungsloch 123 oben herausgezogen ist.

Daraufhin wird ein Fadenende des Fadens 307 aus der Öse 305 entnommen und die Öse 305 vom Faden 307 entfernt. Anschließend werden diese beiden Fadenenden des Fadens 307 beidseitig der Knochenschraube 101 und des Hohlschaftes 203 des Einbringers 201 bis zum proximalen Ende des Einbringers 201 an dessen Handgriff 207 geführt und dort wie oben beschrieben befestigt. Diese manuelle Beladung des ersten Beladungsloches 123 stellt eine weitere Beladungsmöglichkeit zu den oben beschriebenen und in den Figuren 6 bis 8 gezeigten Beladungsmöglichkeiten dar.

Somit wird eine Knochenschraube 101 bereitgestellt, welche aufgrund der Ausführung des ersten Beladungsloches 123 und des zweiten Beladungsloches 125 flexibel sowohl vorbeladen als auch mit unterschiedlichen Beladungsweisen und -konzepten während einer Operation mit einem Faden oder mehreren Fäden beladen werden kann, sodass eine flexibel nutzbare Knochenschraube 101 zum Fixieren eines abgelösten Gewebes an einem Knochen bereitsteht.

### Bezugszeichenliste

- 101: Knochenschraube
- 103: proximales Ende
- 105: distales Ende
- 107: proximaler Körper
- 109: distale Spitze
- 111: Plattenebene
- 113: Längsrichtung
- 115: Gewinde
- 117: Längsbohrung
- 119: proximale Öffnung
- 121: distale Öffnung
- 123: erstes Beladungsloch
- 125: zweites Beladungsloch
- 127: Klemmflügel
- 129: Spaltöffnung
- 131: Spaltbreite
- 133: konischer Freiraum
- 135: Steg
- 137: schräge Anschlagsfläche
- 139: Perforationsloch
- 141: Innensechskantprofil
- 201: Einbringer
- 203: Hohlschaft
- 205: Längsrichtung
- 207: Handgriff
- 209: Nut
- 211: Fadenspule
- 213: Hohlraum
- 215: Schlitz
- 217: Außensechskantprofil
- 301: Einfädler
- 303: Draht
- 305: Öse
- 307: Faden
- 309: Einfädelrichtung

## Patentansprüche

1. Knochenankerelement (101) zum Einbringen in einen Knochen und/oder Fixieren von Gewebe an dem Knochen, wobei das Knochenankerelement (101) in einer Längsrichtung (113) ein proximales Ende (103), einen proximalen Abschnitt (107), einen distalen Abschnitt (109) und ein distales Ende (105) aufweist, und der proximale Abschnitt (107) außen einen profilierten Vorsprung (115) zum Verhindern eines Abziehens des in den Knochen eingebrachten Knochenankerelementes (101) und innen einen durchgehenden Hohlraum (117) in der Längsrichtung (113) mit einer proximalen Öffnung (119) und einer distalen Öffnung (121) aufweist, und der distale Abschnitt (109) plattenförmig abgeflacht mit einer Plattenebene (111) ausgebildet ist, wobei der distale Abschnitt (109) plattenförmig ist und der distale Abschnitt (109) in der Längsrichtung (113) zum distalen Ende (105) einen ersten Querdurchbruch (123) durch die Plattenebene (111) aufweist, wobei der erste Querdurchbruch (123) mit dem durchgehenden Hohlraum (117) des proximalen Abschnittes (107) über die distale Öffnung (121) verbunden ist und anschließend einen zweiten Querdurchbruch (125) durch die Plattenebene (111) aufweist, wobei der erste Querdurchbruch (123) und der zweite Querdurchbruch (125) durch einen Steg (135) des plattenförmigen distalen Abschnittes (109) getrennt sind, und der zweite Querdurchbruch (125) am distalen Ende (105) eine Spaltöffnung (129) in Längsrichtung (113) und im Wesentlichen quer zur Plattenebene (111) aufweist, sodass ein Nahtmaterial (307) flexibel durch den durchgehenden Hohlraum (117), den ersten Querdurchbruch (123), den zweiten Querdurchbruch (125) und/oder die Spaltöffnung (129) führbar ist, und der Steg (135) auf seiner proximalen Seite zum ersten Querdurchbruch (123) eine schräge Anschlagsfläche (137) derart aufweist, dass im Falle eines Durchschiebens einer Öse (305) von der proximalen Öffnung (119) durch den durchgehenden Hohlraum (117) des Knochenankerelementes (101), durch die distale Öffnung (121) und durch den ersten Querdurchbruch (123) in Längsrichtung (113), die Öse (305) beim Auftreffen auf die schräge Anschlagsfläche (137) durch den ersten Querdurchbruch (123) hindurch nach außen geführt wird.

2. Knochenankerelement (101) nach Anspruch 1, **dadurch gekennzeichnet, dass** sich der proximale Abschnitt (107) und/oder der distale Abschnitt (109) stetig oder unstetig vom jeweiligen proximalen Ende zum jeweiligen distalen Ende verjüngt.

3. Knochenankerelement (101) nach einem der vorherigen Ansprüche, **dadurch gekennzeichnet, dass** der erste Querdurchbruch (123) durch die Plattenebene (111) im Wesentlichen viereckig, insbesondere quer zur Längsrichtung (113) rechteckig, ausgebildet ist.

4. Knochenankerelement (101) nach einem der vorherigen Ansprüche, **dadurch gekennzeichnet, dass** der zweite Querdurchbruch (125) durch die Plattenebene (111) im Wesentlichen eine runde Form aufweist.

5. Knochenankerelement (101) nach einem der vorherigen Ansprüche, **dadurch gekennzeichnet, dass** das distale Ende (105) des distalen Abschnittes (109) um die Spaltöffnung (129) in der Plattenebene (111) abgerundet ist und/oder nach der Spaltöffnung (129) einen sich erweiternden Freiraum (133) in Längsrichtung (113) bis zum distalen Ende (105) aufweist.

6. Knochenankerelement (101) nach einem der vorherigen Ansprüche, **dadurch gekennzeichnet, dass** das Knochenankerelement (101) das Nahtmaterial (307) aufweist und eine Spaltbreite (131) der Spaltöffnung (129) in der Plattenebene (111) an einen Durchmesser des Nahtmaterials (307) angepasst und derart eingerichtet ist, dass der Durchmesser des Nahtmaterials (307) durch die Spaltöffnung (129) einfädelbar ist.

7. Knochenankerelement (101) nach einem der vorherigen Ansprüche, **dadurch gekennzeichnet, dass** das Knochenankerelement (101) einstückig ausgebildet ist.

8. Knochenankerelement (101) einem der vorherigen Ansprüche, **dadurch gekennzeichnet, dass** das Knochenankerelement (101) ein biointegrierbares Material aufweist.

9. Knochenankerelement (101) einem der vorherigen Ansprüche, **dadurch gekennzeichnet, dass** der proximale Abschnitt (107) ein Perforationsloch (139) oder mehrere Perforationslöcher aufweist.

10. Knochenankerelement (101) einem der vorherigen Ansprüche, **dadurch gekennzeichnet, dass** die proximale Öffnung (119) ein Innenmitnahmeprofil, insbesondere ein Innensechskantprofil (141), aufweist.

11. Knochenankerelement (101) einem der vorherigen Ansprüche, **dadurch gekennzeichnet, dass** das Knochenankerelement (101) an seinem proximalen Ende (103) und/oder an der proximalen Öffnung (119) ein Verdrehschutzelement aufweist.

12. Knochenankersystem mit einen Knochenankerelement (101) nach einem der Ansprüche 1 bis 11 und einem Einbringer (201) zum Einbringen des Knochenankerelementes in ein Bohrloch in einem Knochen, wobei der Einbringer (201) einen Werkzeugschaft (203) in einer Längsrichtung (205) aufweist, an einem proximalen Ende des Werkzeugschaftes (203) ein Handgriff (207) und in einem gegenüberliegenden, distalen Endabschnitt ein Mitnehmerprofil zum Verbinden mit dem Knochenankerelement (101) angeordnet sind und der Werkzeugschaft (203) zumindest in einem Bereich vor dem distalen Ende bis einschließlich des Mitnehmerprofils einen durchgehenden Hohlraum (213) in der Längsrichtung (205) aufweist, und der Werkzeugschaft (203) vor dem distalen Ende eine Einführöffnung (215) in Längsrichtung (205) aufweist, wobei die Einführöffnung (215) mit dem durchgehenden Hohlraum (213) verbunden ist, sodass der Einbringer (201) mit dem Knochenankerelement (101) verbindbar, von außen eine Öse (305) durch die Einführöffnung (215) in den durchgehenden Hohlraum (213) bis durch das Mitnehmerprofil am distalen Ende des Einbringers (201) und durch den durchgehenden Hohlraum (117) des Knochenankerelementes (101) durch die distale Öffnung (121) in den ersten Querdurchbruch (123) des Knochenankerelementes (101) und über die schräge Anschlagsfläche (137) nach außen über das distale Ende (105) des Knochenankerelementes (101) hinausschiebbar ist, ein Nahtmaterial (307) durch das Knochenankerelement (101) flexibel einfädelbar und das Knochenankerelement (101) mittels des Einbringers (201) in ein Bohrloch in einem Knochen einbringbar ist.

13. Knochenankersystem nach Anspruch 12, **dadurch gekennzeichnet, dass** das Knochenankersystem einen Einfädler (301) mit einer Öse (305) aufweist, sodass im Falle des Verbindens des Einbringers (201) mit dem Knochenankerelement (101) die Öse (305) des Einfädlers (301) von außen durch die Einführöffnung (215) in und durch den Hohlraum (213) des Werkzeugschaftes (203) des Einbringers (201), durch den durchgehenden Hohlraum (213), die distale Öffnung (121) und den ersten Querdurchbruch (123) über die schräge Anschlagsfläche (137) bis nach außen über das distale Ende (105) des Knochenankerelementes (101) schiebbar und in die Öse (305) ein Nahtmaterial (307) einfädelbar ist.

14. Knochenankersystem nach einem der Ansprüche 12 oder 13, **dadurch gekennzeichnet, dass** das Knochenankersystem das Nahtmaterial (307) aufweist.

15. Knochenankersystem nach einem der Ansprüche 12 bis 14, **dadurch gekennzeichnet, dass** das Mitnehmerprofil des Einbringers (201) ein Außenmitnehmerprofil, insbesondere ein Außensechskantprofil (217), aufweist.

16. Knochenankersystem nach einem der Ansprüche 12 bis 15, **dadurch gekennzeichnet, dass** der Einbringer (201) ein Verdrehschutzelement aufweist, sodass im Falle des Verbindens des Einbringers (201) mittels des Mitnehmerprofils mit einem Knochenankerelement (101) ein Verdrehen des verbundenen Knochenankerelementes (101) verhindert ist.

17. Knochenankersystem nach einem der Ansprüche 12 bis 16, **dadurch gekennzeichnet, dass** der Handgriff (207) des Einbringers (201) eine Aussparung (209) und/oder eine Spule (211) zur Aufnahme eines Nahtmaterials aufweist.

18. Verfahren zum Konfektionieren eines Knochenankersystems mittels eines Knochenankersystems nach einem der Ansprüche 12 bis 17, mit folgenden Schritten:
- Verbinden des distalen Endes des Einbringers (201) mit dem proximalen Ende (103) des Knochenankerelementes (101),
- Einführen einer Öse (305) eines Einfädlers (301) von außen durch die Einführöffnung (215) in den Hohlraum (213) des Werkzeugschaftes (203) des Einbringers (201),
- Durchschieben der Öse (305) durch den Hohlraum (213) des Werkzeugschaftes (203) des Einbringers (201) und durch den durchgehenden Hohlraum (117), die distale Öffnung (121) und den ersten Querdurchbruch (123) über die schräge Anschlagsfläche (137) bis nach außen über das distale Ende (105) des Knochenankerelements (101),
- Einfädeln eines Nahtfadens (307) des Nahtmaterials durch die Öse (305),
- Teilweises Zurückziehen des Einfädlers (301), sodass die Öse (305) in Richtung auf das proximale Ende (103) des Knochenankerelements (101) zur Spaltöffnung (129) des zweiten Querdurchbruches (125) verschoben wird,
- Einfädeln des Nahtfadens (307) in die Spaltöffnung (129) des zweiten Querdurchbruches (125),
- Weiteres Zurückziehen des Einfädlers (301), sodass der Nahtfaden (307) an der distalen Seite des Steges (135) anliegt,
- Ziehen eines ersten Endes des Nahtfadens (307) aus der Öse (305) und Führen des ersten Endes entlang einer Außenseite des Knochenankerelements (101) bis über sein proximales Ende (103),
- vollständiges Zurückziehen des Einfädlers (301) aus der Einführöffnung (215) des Werkzeugschaftes (203) heraus, sodass ein zweites Ende des Nahtfadens (307) über den Steg (135) und durch den durchgehenden Hohlraum (117) des proximalen Abschnittes (107) bis über das proximale Ende (103) des Knochenankerelements (101) aus der Einführöffnung (215) herausgezogen wird, und
- Festhalten oder Befestigen der beiden Enden des Nahtfadens (307) an dem Handgriff (207) des Einbringers (201),
sodass der Nahtfaden knotenlos am Knochenankerelement befestigt ist.

## Claims

1. Bone anchor element (101) for inserting into a bone and/or fixing tissue to the bone, wherein the bone anchor element (101) has in a longitudinal direction (113) a proximal end (103), a proximal section (107), a distal section (109) and a distal end (105), and the proximal section(107) has on the outside a contoured projection (115) to prevent the bone anchor element (101) inserted into the bone from being pulled out and on the inside has a continuous hollow space (117) in the longitudinal direction (113) having a proximal opening (119) and a distal opening (121), and the distal section(109) is formed in a flattened, plate-shaped manner having a plate plane (111), wherein the distal section (109) is plate-shaped and the distal section (109) in the longitudinal direction (113) to the distal end (105) has a first transverse through-hole (123) through the plate plane (111), wherein the first transverse through-hole (123) is connected to the continuous hollow space (117) of the proximal section (107) via the distal opening (121) and subsequently has a second transverse through-hole (125) through the plate plane (111), wherein the first transverse through-hole (123) and the second transverse through-hole (125) are separated by a web (135) of the plate-shaped distal section (109), and the second transverse through-hole (125) has on the distal end(105) a gap opening (129) in the longitudinal direction (113) and essentially transverse to the plate plane (111), such that a suture material (307) can be flexibly guided through the continuous hollow space (117), the first transverse through-hole (123), the second transverse through-hole (125) and/or the gap opening (129), and on its proximal side in relation to the first transverse through-hole (123) the web (135) has an inclined abutment surface (137) such that in the event that an eyelet (305) is pushed through from the proximal opening (119) through the continuous space (117) of the bone anchor element (101), through the distal opening (121) and through the first transverse through-hole (123) in the longitudinal direction (113), the eyelet (305) is guided outwards through the first transverse through-opening (123) upon striking the inclined abutment surface (137).

2. Bone anchor element (101) according claim 1, **characterised in that** the proximal section (107) and/or the distal section (109) tapers continuously or non-continuously from the respective proximal end to the respective distal end.

3. Bone anchor element (101) according to any one of the preceding claims, **characterised in that** the first transverse through-hole (123) through the plate plane (111) is formed essentially quadrilaterally, in particular rectangularly transverse to the longitudinal direction (113).

4. Bone anchor element (101) according to any one of the preceding claims, **characterised in that** the second transverse through-hole (125) through the plate plane (111) has an essentially round shape.

5. Bone anchor element (101) according to any one of the preceding claims, **characterised in that** the distal end (105) of the distal section (109) is rounded about the gap opening (129) in the plate plane (111) and/or after the gap opening (129) has a clearance space (133) widening in the longitudinal direction (113) to the distal end (105).

6. Bone anchor element (101) according to any one of the preceding claims, **characterised in that** the bone anchor element (101) has the suture material (307) and a gap width (131) of the gap opening (129) in the plate plane (111) is adapted to a diameter of the suture material (307) and configured in such a way that the diameter of the suture material (307) can be threaded through the gap opening (129).

7. Bone anchor element (101) according to any one of the preceding claims, **characterised in that** the bone anchor element (101) is designed in one piece.

8. Bone anchor element (101) according to any one of the preceding claims, **characterised in that** the bone anchor element (101) has a biointegratable material.

9. Bone anchor element (101) according to any one of the preceding claims, **characterised in that** the proximal end (107) has a perforation (139) or a plurality of perforations.

10. Bone anchor element (101) according to any one of the preceding claims, **characterised in that** the proximal opening (119) has an internal driving profile, in particular an internal hexagonal profile (141).

11. Bone anchor element (101) according to any one of the preceding claims, **characterised in that** on its proximal end (103) and/or at the proximal opening (119) the bone anchor element (101) has an anti-rotation protection element.

12. Bone anchor system having a bone anchor element (101) according to any one of claims 1 to 11 and an inserter (201) for inserting the bone anchor element into a drill hole in a bone, wherein the inserter (201) has a tool shaft (203) in a longitudinal direction (205), a handle (207) is arranged at a proximal end of the tool shaft (203) and a driving profile for connection to the bone anchor element (101) is arranged at an opposing distal end section, and at least in one region before the distal end up to and including the driving profile the tool shaft (203) has a continuous hollow profile (213) in the longitudinal direction (205), and before the distal end the tool shaft (203) has an insertion opening (215) in the longitudinal direction (205), wherein the insertion opening (215) is connected to the continuous hollow space (213) such that the inserter (201) can be connected to the bone anchor element (101), an eyelet (305) can be pushed from the outside through the insertion opening (215) into the continuous hollow space (213) all the way through the driving profile on the distal end of the inserter (201) and through the continuous hollow space (117) of the bone anchor element (101) through the distal opening (121) into the first transverse through-hole (123) of the bone anchor element (101) and via the inclined abutment surface (137) outwards over the distal end (105) of the bone anchor element (101), a suture material (307) can be flexibly threaded through the bone anchor element (101) and the bone anchor element (101) can be introduced into a drill hole in a bone by means of the inserter (201).

13. Bone anchor system according to claim 12, **characterised in that** the bone anchor system has a threader (301) with an eyelet (305) such that in the event of connecting the inserter (201) to the bone anchor element (101), the eyelet (305) of the threader (301) can be pushed from the outside through the insertion opening (215) into and through the hollow space (213) of the tool shaft (203) of the inserter (201), through the continuous hollow space (213), the distal opening (121) and the first transverse through-hole (123) via the inclined abutment surface (137) to the outside over the distal end (105) of the bone anchor element (101) and a suture material (307) can be threaded into the eyelet (305).

14. Bone anchor system according to any one of claims 12 or 13, **characterised in that** the bone anchor system has the suture material (307).

15. Bone anchor system according to any one of claims 12 to 14, **characterised in that** the driving profile of the inserter (201) has an external driving profile, in particular an external hexagonal profile (217).

16. Bone anchor system according to any one of claims 12 to 15, **characterised in that** the inserter (201) has an anti-rotation protection element such that in the event of connecting the inserter (201) by means of the driving profile to the bone anchor element (101), rotation of the connected bone anchor element (101) is prevented.

17. Bone anchor system according to any one of claims 12 to 16, **characterised in that** the handle (207) of the inserter (201) has a recess (209) and/or a spool (211) for receiving a suture material.

18. Method for assembling a bone anchor system using a bone anchor system according to any one of claims 12 to 17, having the following steps:
- connecting the distal end of the inserter (201) to the proximal end (103) of the bone anchor element (101),
- inserting an eyelet (305) of a threader (301) from the outside through the insertion opening (215) in the hollow space (213) of the tool shaft (203) of the inserter (201),
- pushing the eyelet (305) through the hollow space (213) of the tool shaft (203) of the inserter (201) and through the continuous hollow space (117), the distal opening (121) and the first transverse through-opening (123) over the inclined abutment surface (137) to the outside beyond the distal end (105) of the bone anchor element (101),
- threading a suture thread (307) of the suture material through the eyelet (305),
- partially retracting the threader (301) such that the eyelet (305) is displaced in the direction of the proximal end (103) of the bone anchor element (101) towards the gap opening (129) of the second transverse through-hole (125),
- threading the suture thread (307) into the gap opening ( 129) of the second transverse through-hole (125),
- further retracting the threader (301) such that the suture thread (307) contacts the distal side of the web (135),
- pulling a first end of the suture thread (307) out of the eyelet (305) and guiding the first end along an exterior side of the bone anchor element (101) and over its proximal end (103),
- completely retracting the threader (301) out of the insertion opening (215) of the tool shaft (203) such that a second end of the suture thread (307) is guided over the web (135) and through the continuous hollow space (117) of the proximal section (107) to beyond the proximal end (103) of the bone anchor element (101) and out of the insertion opening (215), and
- holding or attaching the two ends of the suture thread (307) to the handle (207) of the inserter (201) such that the suture thread is attached to the bone anchor element without a knot.

## Revendications

1. Elément d'ancrage osseux (101) destiné à être inséré dans un os et/ou destiné à fixer un tissu sur l'os, dans lequel l'élément d'ancrage osseux (101) présente dans une direction longitudinale (113) une extrémité proximale (103), une section proximale (107), une section distale (109) et une extrémité distale (105), et la section proximale (107) présente à l'extérieur une partie faisant saillie profilée (115) destinée à empêcher un retrait de l'élément d'ancrage osseux (101) introduit dans l'os et à l'intérieur un espace creux (117) traversant dans la direction longitudinale (113) avec une ouverture proximale (119) et une ouverture distale (121), et la section distale (109) est réalisée de manière aplatie en forme de panneau avec un plan de panneau (111),
dans lequel la section distale (109) est en forme de panneau et la section distale (109) présente dans la direction longitudinale (113) vers l'extrémité distale (105) un premier ajour transversal (123) à travers le plan de panneau (111), dans lequel le premier ajour transversal (123) est relié à l'espace creux (117) traversant de la section proximale (107) par l'intermédiaire de l'ouverture distale (121) puis présente un deuxième ajour transversal (125) à travers le plan de panneau (111), dans lequel le premier ajour transversal (123) et le deuxième ajour transversal (125) sont séparés par une entretoise (135) de la section distale (109) en forme de panneau, et le deuxième ajour transversal (125) présente sur l'extrémité distale (105) une ouverture de fente (129) dans la direction longitudinale (113) et de manière sensiblement transversale par rapport au plan de panneau (111) si bien qu'un matériau de suture (307) peut être guidé de manière flexible à travers l'espace creux (117) traversant, le premier ajour transversal (123), le deuxième ajour transversal (125) et/ou l'ouverture de fente (129), et l'entretoise (135) présente, sur son côté proximal vers le premier ajour transversal (123), une surface de butée oblique (137) de telle manière qu'en cas de glissement d'un œillet (305) depuis l'ouverture proximale (119) à travers l'espace creux (117) traversant de l'élément d'ancrage osseux (101), par l'ouverture distale (121) et par le premier ajour transversal (123) dans la direction longitudinale (113), l'œillet (305) est guidé vers l'extérieur à travers le premier ajour transversal (123) de part en part lorsqu'il heurte la surface de butée oblique (137).

2. Elément d'ancrage osseux (101) selon la revendication 1, **caractérisé en ce que** la section proximale (107) et/ou la section distale (109) se rétrécissent de manière continue ou de manière discontinue depuis l'extrémité proximale respective vers l'extrémité distale respective.

3. Elément d'ancrage osseux (101) selon l'une quelconque des revendications précédentes, **caractérisé en ce que** le premier ajour transversal (123) est réalisé à travers le plan de panneau (111) sensiblement de manière quadrangulaire, en particulier de manière rectangulaire de manière transversale par rapport à la direction longitudinale (113).

4. Elément d'ancrage osseux (101) selon l'une quelconque des revendications précédentes, **caractérisé en ce que** le deuxième ajour transversal (125) présente à travers le plan de panneau (111) sensiblement une forme ronde.

5. Elément d'ancrage osseux (101) selon l'une quelconque des revendications précédentes, **caractérisé en ce que** l'extrémité distale (105) de la section distale (109) est arrondie autour de l'ouverture de fente (129) dans le plan de panneau (111) et/ou présente après l'ouverture de fente (129) un espace libre (133) s'élargissant dans la direction longitudinale (113) jusqu'à l'extrémité distale (105).

6. Elément d'ancrage osseux (101) selon l'une quelconque des revendications précédentes, **caractérisé en ce que** l'élément d'ancrage osseux (101) présente le matériau de suture (307) et une largeur de fente (131) de l'ouverture de fente (129) est adaptée dans le plan de panneau (111) à un diamètre du matériau de suture (307) et est mise au point de telle manière que le diamètre du matériau de suture (307) peut être enfilé à travers l'ouverture de fente (129).

7. Elément d'ancrage osseux (101) selon l'une quelconque des revendications précédentes, **caractérisé en ce que** l'élément d'ancrage osseux (101) est réalisé d'un seul tenant.

8. Elément d'ancrage osseux (101) selon l'une quelconque des revendications précédentes, **caractérisé en ce que** l'élément d'ancrage osseux (101) présente un matériau biointégrable.

9. Elément d'ancrage osseux (101) selon l'une quelconque des revendications précédentes, **caractérisé en ce que** la section proximale (107) présente un trou de perforation (139) ou plusieurs trous de perforation.

10. Elément d'ancrage osseux (101) selon l'une quelconque des revendications précédentes, **caractérisé en ce que** l'ouverture proximale (119) présente un profil d'entraînement intérieur, en particulier un profil à six pans creux (141).

11. Elément d'ancrage osseux (101) selon l'une quelconque des revendications précédentes, **caractérisé en ce que** l'élément d'ancrage osseux (101) présente sur son extrémité proximale (103) et/ou sur l'ouverture proximale (119) un élément de protection anti-rotation.

12. Système d'ancrage osseux avec un élément d'ancrage osseux (101) selon l'une quelconque des revendications 1 à 11 et un dispositif d'introduction (201) destiné à introduire l'élément d'ancrage osseux dans un trou de forage dans un os, dans lequel le dispositif d'introduction (201) présente une tige d'outil (203) dans une direction longitudinale (205), une poignée (207) est disposée sur une extrémité proximale de la tige d'outil (203) et un profil d'entraînement destiné à être relié à l'élément d'ancrage osseux (101) est disposé dans une section d'extrémité distale opposée et la tige d'outil (203) présente au moins dans une zone devant l'extrémité distale jusqu'au profil d'entraînement inclus un espace creux (213) traversant dans la direction longitudinale (205), et la tige d'outil (203) présente une ouverture d'insertion (215) dans la direction longitudinale (205) devant l'extrémité distale, dans lequel l'ouverture d'insertion (215) est reliée à l'espace creux (213) traversant si bien le dispositif d'introduction (201) peut être relié à l'élément d'ancrage osseux (101), un œillet (305) peut être sorti par glissement depuis l'extérieur à travers l'ouverture d'insertion (215) dans l'espace creux (213) traversant jusqu'à travers le profil d'entraînement sur l'extrémité distale du dispositif d'introduction (201) et à travers l'espace creux (117) traversant de l'élément d'ancrage osseux (101), à travers l'ouverture distale (121) dans le premier ajour transversal (123) de l'élément d'ancrage osseux (101) et au-dessus de la surface de butée oblique (137) vers l'extérieur au-delà de l'extrémité distale (105) de l'élément d'ancrage osseux (101), un matériau de suture (307) peut être enfilé de manière flexible à travers l'élément d'ancrage osseux (101) et l'élément d'ancrage osseux (101) peut être introduit dans un trou de forage dans un os au moyen du dispositif d'introduction (201).

13. Système d'ancrage osseux selon la revendication 12, **caractérisé en ce que** le système d'ancrage osseux présente un dispositif d'enfilage (301) avec un œillet (305) si bien que lorsque le dispositif d'introduction (201) est relié à l'élément d'ancrage osseux (101), l'œillet (305) du dispositif d'enfilage (301) peut être glissé depuis l'extérieur à travers l'ouverture d'insertion (215) dans et à travers l'espace creux (213) de la tige d'outil (203) du dispositif d'introduction (201), à travers l'espace creux (213) traversant, l'ouverture distale (121) et le premier ajour transversal (123) au-dessus de la surface de butée oblique (137) jusque vers l'extérieur au-delà de l'extrémité distale (105) de l'élément d'ancrage osseux (101) et un matériau de suture (307) peut être enfilé dans l'œillet (305).

14. Système d'ancrage osseux selon l'une quelconque des revendications 12 ou 13, **caractérisé en ce que** le système d'ancrage osseux présente le matériau de suture (307).

15. Système d'ancrage osseux selon l'une quelconque des revendications 12 à 14, **caractérisé en ce que** le profil d'entraînement du dispositif d'introduction (201) présente un profil d'entraînement extérieur, en particulier un profil à six pans creux extérieur (217).

16. Système d'ancrage osseux selon l'une quelconque des revendications 12 à 15, **caractérisé en ce que** le dispositif d'introduction (201) présente un élément de protection anti-rotation si bien que lorsque le dispositif d'introduction (201) est relié à un élément d'ancrage osseux (101) au moyen du profil d'entraînement, une rotation de l'élément d'ancrage osseux (101) relié est empêchée.

17. Système d'ancrage osseux selon l'une quelconque des revendications 12 à 16, **caractérisé en ce que** la poignée (207) du dispositif d'introduction (201) présente un évidement (209) et/ou une bobine (211) destinée à recevoir un matériau de suture.

18. Procédé de fabrication d'un système d'ancrage osseux au moyen d'un système d'ancrage osseux selon l'une quelconque des revendications 12 à 17, avec des étapes suivantes :
- la liaison de l'extrémité distale du dispositif d'introduction (201) à l'extrémité proximale (103) de l'élément d'ancrage osseux (101),
- l'insertion d'un œillet (305) d'un dispositif d'enfilage (301) depuis l'extérieur à travers l'ouverture d'insertion (215) dans l'espace creux (213) de la tige d'outil (203) du dispositif d'introduction (201),
- le passage par glissement de l'œillet (305) à travers l'espace creux (213) de la tige d'outil (203) du dispositif d'introduction (201) et à travers l'espace creux (117) traversant, l'ouverture distale (121) et le premier ajour transversal (123) au-dessus de la surface de butée oblique (137) jusque vers l'extérieur au-delà de l'extrémité distale (105) de l'élément d'ancrage osseux (101),
- l'enfilage d'un fil de suture (307) du matériau de suture à travers l'œillet (305),
- le retrait partiel du dispositif d'enfilage (301), si bien que l'œillet (305) est déplacé par coulissement en direction de l'extrémité proximale (103) de l'élément d'ancrage osseux (101) jusqu'à l'ouverture de fente (129) du deuxième ajour transversal (125),
- l'enfilage du fil de suture (307) dans l'ouverture de fente (129) du deuxième ajour transversal (125),
- le retrait ultérieur du dispositif d'enfilage (301) si bien que le fil de suture (307) repose sur le côté distal de l'entretoise (135),
- le tirage d'une première extrémité du fil de suture (307) hors de l'œillet (305) et le guidage de la première extrémité le long d'un côté extérieur de l'élément d'ancrage osseux (101) jusqu'au-delà de son extrémité proximale (103),
- le retrait total du dispositif d'enfilage (301) hors de l'ouverture d'insertion (215) de la tige de l'outil (203), si bien qu'une seconde extrémité du fil de suture (307) est retirée en passant par l'entretoise (135) et à travers l'espace creux (117) traversant de la section proximale (107) jusqu'au-delà de l'extrémité proximale (103) de l'élément d'ancrage osseux (101) hors de l'ouverture d'insertion (215), et
- le maintien ou la fixation des deux extrémités du fil de suture (307) sur la poignée (207) du dispositif d'introduction (201)
si bien que le fil de suture est fixé sans noeud sur l'élément d'ancrage osseux.
